# EUROPEAN PATENT APPLICATION

(11) **EP 1 757 235 A1**
(43) Date of publication of application: **28.02.2007**
(21) Application number: 06076656.5
(22) Date of filing: 25.08.2006
(51) Int. Cl.: A61B 17/10, A61B 17/122

(54) **Surgical jaw control device**

(30) Priority: 25.08.2005 US 210830
(71) Applicant: Microline Pentax Inc., Beverly MA 01915 (US)
(72) Inventor: Aliski, Peter, Malden MA Massachusetts 02148 (US); Menn, Pavel, Marblehead MA Massachusetts 01945 (US); Theroux, Marc, Grafton MA Massachusetts 01519 (US)
(74) Representative: Schaumburg, Thoenes, Thurn, Landskron

(57) **Abstract**

A mechanism for opening and closing jaws of a medical instrument. The mechanism includes a cinch configured to selectively move in opposite directions along a longitudinal axis, the cinch comprising a pair of control members each having an inner control surface and an outer control surface, and a jaw assembly having a pair of jaw arms, each jaw arm comprising an inner engagement surface configured to slidingly engage with a respective the outer control surface, and an outer engagement surface configured to slidingly engage with a respective inner control surface.

## Description

### BACKGROUND OF THE INVENTION

### 1. Field of the Invention

The present invention is related to a cinch control, and more particularly relates to a cinch control device for medical clip applying device.

### 2. Background and Material Information

Laparoscopic surgery is generally defined as minimally invasive surgery upon a patient, utilizing small or miniaturized medical devices by which body tissue is cut, removed or cauterized by small manipulable tools/devices through small incisions or openings within the patient's body. One such tool is a clip applier, which is used to grasp and/or crimp/seal tissue by the single hand of an operating surgeon., and is described in commonly-assigned U.S. Patent Publication No. 2003/0040759 and U.S. Patent No. 6,277,131, the entire contents of both documents being expressly incorporated by reference herein.

Prior art clip appliers have a patient-engaging distalmost end with a pair of squeezable jaws arranged on the distal end of an elongated channel or frame. The elongated channel is surrounded by an elongated tube, which elongated tube and elongated channel are secured at their respective proximalmost ends to the distal end of a pistol-like handle grip assembly. The handle grip assembly includes an arcuately movable squeezable trigger. By squeezing the trigger towards a housing portion of the handle grip assembly, a clip is advanced through the elongated channel and into the jaws from an elongated ladder-like clip supply cartridge disposed through the elongated housing. The actuating sequence includes the squeezing of the trigger to close the jaws and thus crimp the clip between the jaws, then releasing the trigger to advance a new clip into location between the jaws awaiting the next squeezing of the trigger. The elongated clip supply cartridge is fed into a receiving slot or port in the proximal end of the handle grip assembly. Once all of the clips have been discharged from the cartridge, the cartridge may be removed from the clip applier and discarded, and the clip applier may be sterilized and reused.

It is also noted that manufacturers of cartridges for reusable clip appliers typically produce different clip supply cartridges each having a differently sized and/or shaped set of clips for use with different clip appliers. Depending on the type of surgery, the surgeon may specify a clip of a specific size, ranging from small to large. Sizes may vary depending on factors such as width (*i.e.*, the gap between the prongs of the clip) and gauge of material. Only a single-sized series of clips may fit into a specific cartridge: if the clips are too small, then they cannot be securely held within the cartridge, and if the clips are too large, then they will not fit within the confines of the cartridge.

For example, it is also known that a cartridge containing medium-sized clips can be used only with a medium-sized clip applier that accepts medium-sized clip cartridges, and the medium-sized clip applier can only hold medium-sized clip cartridges. Similarly, a cartridge containing large-sized clips can be used only with a large-sized clip applier that accepts large-sized clip cartridges, and the large-sized clip applier can only hold large-sized clips. For example, if the cartridge is too small, then the cartridge cannot be securely held within receiving slot or port, and if the cartridge is too large, then it will not fit within the cartridge.

Therefore, if the nature of a surgeon's practice dictates that different sized clips be used for various surgical procedures, then a surgeon must keep an inventory of differently sized clip appliers, which results in increased cost and inventory time. Additionally, if a single surgical procedure requires the use of two different sized clips, then the surgeon must use two different clip appliers, and switching between clip appliers is a time-consuming process. Therefore, a need has arisen for a clip applier that can accept and crimp differently-sized and/or shaped clips and without increased cinch travel.

### SUMMARY OF THE INVENTION

A feature of the present invention provides a clip applying device that is able to accept two different types of clip cartridges. With such a configuration, clip applying jaws move radially over a greater distance than other clip appliers, such full, positive and consistent control of the jaws is required. Further, limited travel in the axial direction X of a cinch is normally not enough to cover this increased radial travel of the jaws. If cinch travel in the axial direction X is increased, then the surgeon may have difficulty operating the device, since the device may be too long for precise control. Thus, there is no single surface of the cinch or jaw assembly to completely and accurately control the opening and closing of the cinch, when using a cinch with reduced travel.

A non-limiting embodiment of the present invention provides a mechanism for opening and closing jaws of a medical instrument. The mechanism may include a cinch configured to selectively move in opposite directions along a longitudinal axis, the cinch having a pair of control members each having an inner control surface and an outer control surface, and a jaw assembly having a pair of jaw arms, each jaw arm having an inner engagement surface configured to slidingly engage with a respective the outer control surface, and an outer engagement surface configured to slidingly engage with a respective inner control surface.

In another feature, when the cinch moves rearwardly from an intermediate position, the inner engagement surfaces slidingly engage with respective outer control surfaces to move the pair of jaw arms apart. In a further feature, when the cinch moves forwardly from an intermediate position toward a closed jaw position, the outer engagement surfaces respectively slidingly engage with the inner control surfaces to move the pair of jaw arms toward each other.

In an additional feature, each inner engagement surface and outer engagement surface are discontinuous with each other. Also, each jaw arm may include a second inner engagement surface, and the cinch may include a central block having opposed ends, each end of the opposed ends configured to contact a respective second inner engagement surface to open the jaw arms when the cinch moves rearwardly along the longitudinal axis.

In another feature, each inner engagement surface and each outer engagement surface are located on the same surface of a respective jaw arm, and each second inner engagement surface are located on a surface of a respective jaw arm that is opposite to the jaw arm surface on which each inner engagement surface and each outer engagement surface are located.

Also, the pair of control members may be a pair of control fingers, and a leading edge of each control finger of the pair of control fingers may be affixed to a distal end of the cinch and forms the inner control surface, and a trailing edge of each control finger may form the outer control surface. Further, at least a portion of each jaw arm may be sandwiched between an underside of the cinch and a respective control finger.

In an additional feature, the cinch further includes a pair of inwardly facing guide walls configured to respectively slidingly engage at least a portion of an outer side region of the pair of jaw arms during movement of the cinch along the longitudinal axis. Also, the jaw arms may be biased to an open position.

A feature of the invention further provides a method of opening and closing jaws of a medical instrument. The instrument may include a cinch movable between a first position and a second position, and to an intermediate position located between the first and second positions, the cinch having a pair of control members, each control member having an inner control surface and an outer control surface, the instrument further including a jaw assembly having a pair of jaw arms, each jaw arm comprising an inner engagement surface and an outer engagement surface. The method may include moving the cinch along a longitudinal axis of the instrument, from the intermediate position toward the first position such that the inner engagement surfaces slidingly engage with the respective outer control surfaces to one of move the jaws together or move the jaws apart, and moving the cinch along the axis from the intermediate position toward the second position such that the outer engagement surfaces slidingly engage with the respective inner control surfaces to the other of move the jaws together or move the jaws apart.

The method may further include moving the cinch along the axis in one of the first or second directions such that each opposed end of the central block contacts a respective second inner engagement surface to open the jaw arms.

In another feature, each inner engagement surface and each outer engagement surface are located on the same surface of a respective jaw arm, and each second inner engagement surface are located on a surface of a respective jaw arm that is opposite to the jaw arm surface on which each inner engagement surface and each outer engagement surface are located. Also, at least a portion of each jaw arm may be sandwiched between an underside of the cinch and a respective control finger.

In another feature, the cinch may further include a pair of inwardly facing guide walls, and the method may further include moving the cinch along the axis such that at least a portion of respective outer side regions of the pair of jaw arms respectively slidingly engage the pair of guide walls. In still another feature, the first position may be a proximal position, the second position may be a distal position, the moving of the cinch from the intermediate position toward the first position moves the jaws apart, and the moving of the cinch from the intermediate position toward the second position moves the jaws together.

Another feature provides a clip applying device for crimping a clip onto tissue. The device may have a body assembly having a handle and a squeezable trigger, a barrel having a first end extending into the body assembly, a cinch configured to move rearwardly and forwardly along a longitudinal axis, the cinch having a pair of control members each having an inner control surface and an outer control surface, a pair of jaw arms arranged on a second end of the barrel, the jaws actuable by movement of the cinch in response to action of the trigger, each jaw arm having an inner engagement surface configured to slidingly engage with a respective outer control surface, and an outer engagement surface configured to slidingly engage with a respective inner control surface, the second end of the barrel and at least a portion of the pair of jaw arms configured to be inserted into a body cavity.

In another feature, each jaw arm may further have a second inner engagement surface, and the cinch may have a central block having opposed ends, each end of the opposed ends configured to contact a respective second inner engagement surface to open the jaw arms when the cinch moves rearwardly along the longitudinal axis.

Other exemplary embodiments and advantages of the present invention may be ascertained by reviewing the present disclosure and the accompanying drawings, and the above description should not be considered to limit the scope of the present invention.

### BRIEF DESCRIPTION OF THE DRAWINGS

The present invention is further described in the detailed description which follows, in reference to the noted plurality of drawings, by way of non-limiting examples of preferred embodiments of the present invention, in which like characters represent like elements throughout the several views of the drawings, and wherein:
Fig. 1 is a sectional view of a medical clip applying device according to an embodiment of the present invention, showing a large cartridge inserted therein;
Fig. 2 is a bottom plan view of a distal end of the medical clip applying device, showing a medium-large clip inserted therein;
Fig. 3 is a bottom plan view of a distal end of the medical clip applying device, showing a large clip inserted therein;
Fig. 4 is a perspective view of a medium-large clip supply cartridge for use with an embodiment of the present invention;
Fig. 5 is a perspective view of a large clip supply cartridge for use with an embodiment of the present invention;
Fig. 6 is a side sectional view of an elongated tube of an embodiment of the present invention, showing a medium-large clip supply cartridge inserted therein;
Fig. 7 is another side sectional view of an elongated tube of an embodiment of the present invention, showing a medium-large clip supply cartridge inserted therein;
Fig. 8 is a side sectional view of an elongated tube of an embodiment of the present invention, showing a large clip supply cartridge inserted therein;
Fig. 9 is another side sectional view of an elongated tube of an embodiment of the present invention, showing a large clip supply cartridge inserted therein;
Fig. 10 is a perspective view of a jaw assembly and cinch of an embodiment of the present invention;
Fig. 11 is a perspective view of the underside of a jaw assembly of an embodiment of the present invention;
Fig. 12 is an enlarged sectional view of a handle grip assembly of an embodiment of the present invention;
Fig.13 is a perspective view of the underside of a cinch of an embodiment of the present invention;
Fig. 14 is a perspective view of the underside of the cinch in an intermediate position;
Fig. 15 is a plan view of the underside of the cinch of an embodiment of the present invention, in a proximal position;
Fig.16 is a top plan view of the of the cinch of an embodiment of the present invention, in the proximal position;
Fig. 17 is a plan view of the underside of the cinch and jaw assembly of an embodiment of the present invention, when the jaws are in a partially-closed position;
Fig. 18 is a plan view of the jaw assembly and cinch of an embodiment of the present invention, when the jaws are in a closed position;
Fig. 19 is an isometric view of the jaw assembly of an embodiment of the present invention;
Fig. 20 is an upper isometric view of the cinch of an embodiment of the present invention;
Fig. 21 is a lower isometric view of the cinch of an embodiment of the present invention;
Fig. 22 is a side elevational view of the jaw assembly;
Fig. 23 is a perspective view of a cartridge alignment device of an embodiment of the present invention;
Fig. 24 is a frontal view of the cartridge alignment device of an embodiment of the present invention;
Fig. 25 is another perspective view of the cartridge alignment device of an embodiment of the present invention;
Fig. 26 is a bottom perspective view of the cartridge alignment device of an embodiment of the present invention;
Fig. 27 is a perspective, sectional view of a slider of an embodiment of the present invention;
Fig. 28 is a front cross-sectional view of the elongated tube taken along the lines of 28-28 in Fig. 6;
Fig. 29 is a sectional view of a slider toggle in an upright position;
Fig. 30 is an enlarged sectional view of the slider toggle shown in Fig. 29;
Fig. 31 is a sectional view of the slider toggle in a lowered position;
Fig. 32 is an enlarged sectional view of the slider shown in Fig. 31;
Fig. 33 is a bottom plan view of the slider;
Fig. 34 is a top plan view of the slider;
Fig. 35 is a perspective view of the underside of the slider;
Fig. 36 is a perspective view of the top of the slider;
Fig. 37 is a bottom plan view of the slider attached to an underside of a spine and in a distally-moved position;

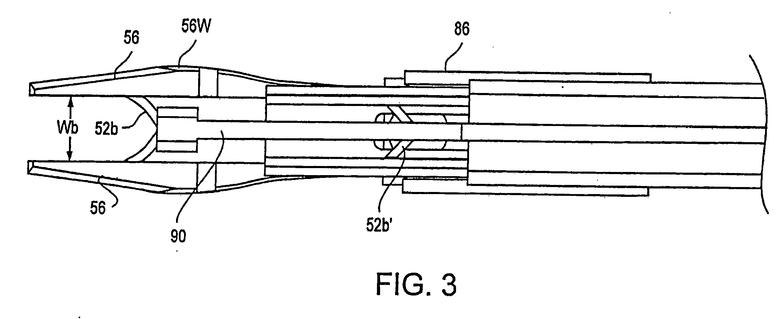

Fig. 3 8 is a bottom perspective view of the slider attached to an underside of a spine and in a distally-moved position;

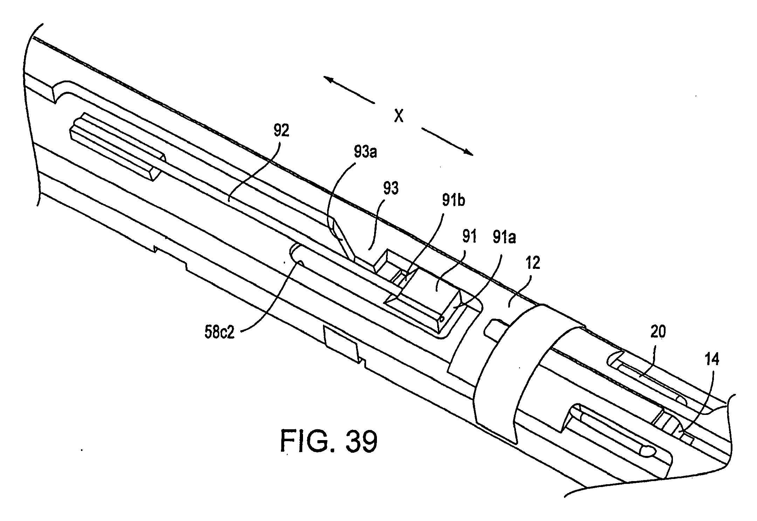

Fig. 39 is a bottom perspective view of the slider attached to an underside of a spine and in a proximally-moved position;

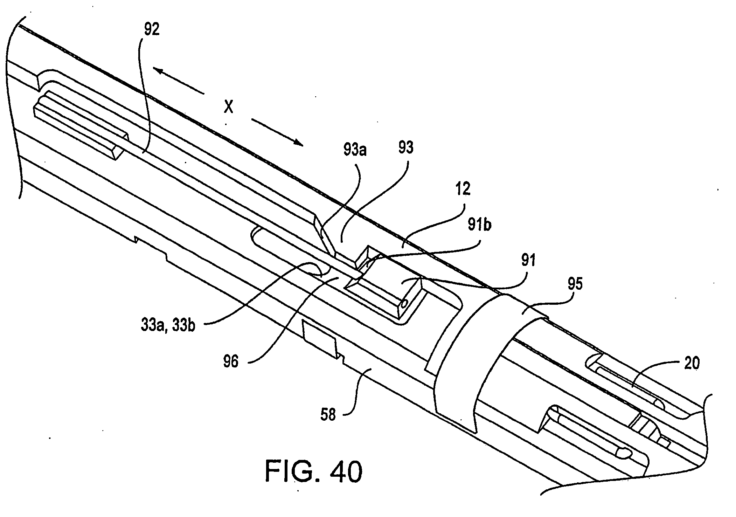

Fig. 40 is a bottom perspective view of the slider attached to an underside of a spine and in a proximally-moved and locked position;

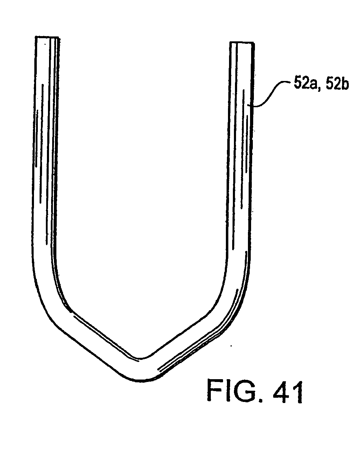

Fig. 41 is a plan view of an uncrimped clip for use in an embodiment of the present invention;

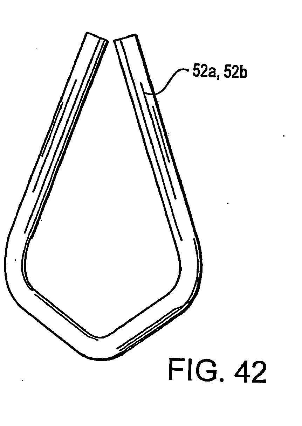

Fig. 42 is a plan view of a partially crimped clip for use in an embodiment of the present invention;

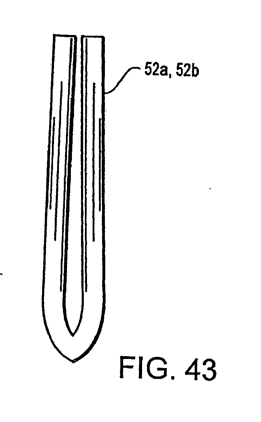

Fig. 43 is a plan view of a generally fully crimped clip for use in an embodiment of the present invention;

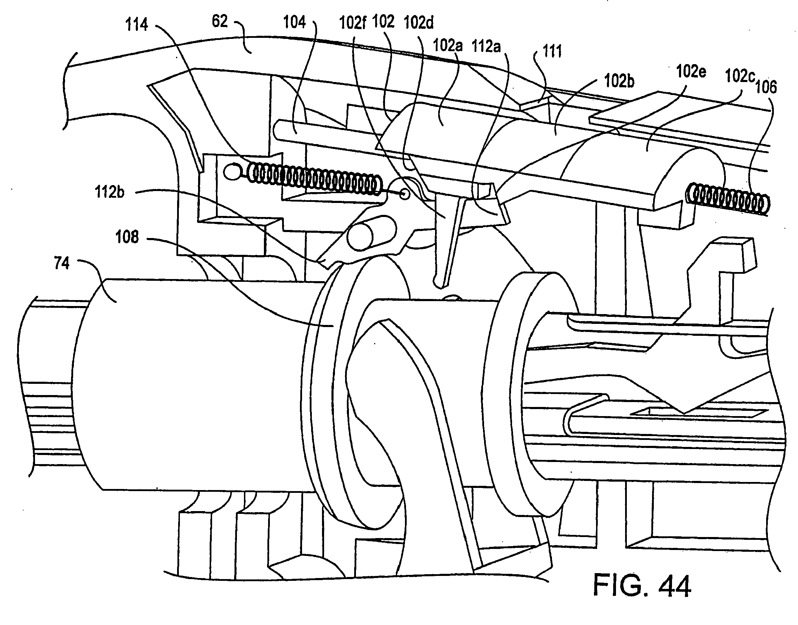

Fig. 44 is an enlarged perspective sectional view of an indicating arrangement according to an embodiment of the present invention in a partially-crimped position; and

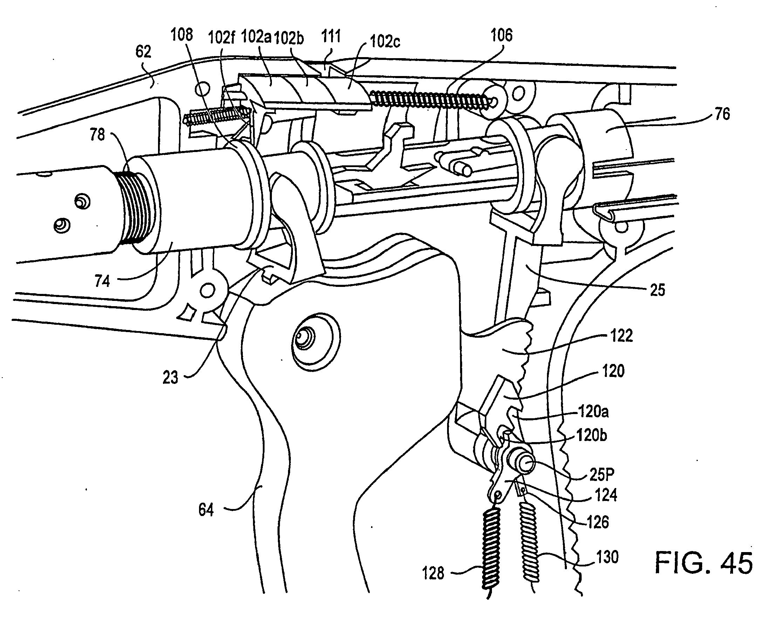

Fig. 45 is an enlarged perspective sectional view of an indicating arrangement according to an embodiment of the present invention in a generally fully-crimped position.

### DETAILED DESCRIPTION OF THE PREFERRED EMBODIMENTS

The particulars shown herein are by way of example and for purposes of illustrative discussion of the embodiments of the present invention only and are presented in the cause of providing what is believed to be the most useful and readily understood description of the principles and conceptual aspects of the present invention. In this regard, no attempt is made to show structural details of the present invention in more detail than is necessary for the fundamental understanding of the present invention, the description taken with the drawings making apparent to those skilled in the art how the several forms of the present invention may be embodied in practice.

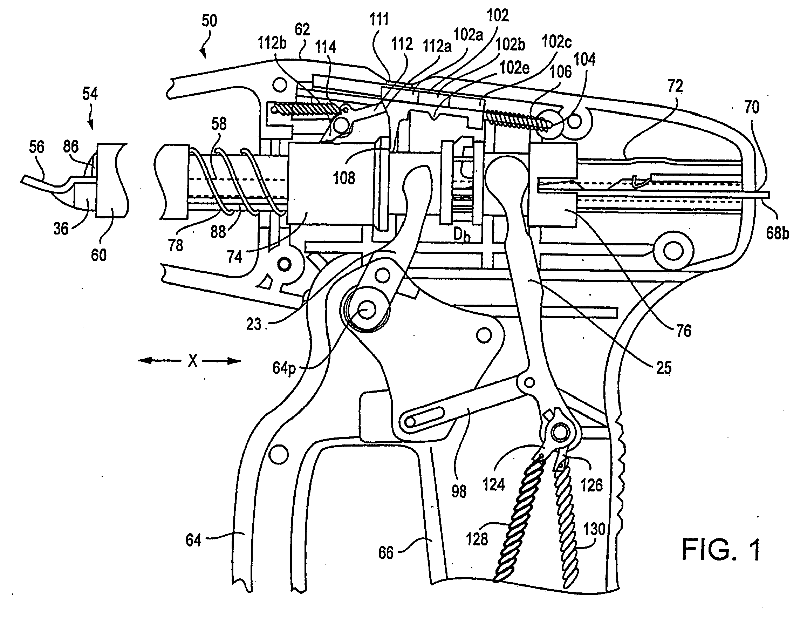

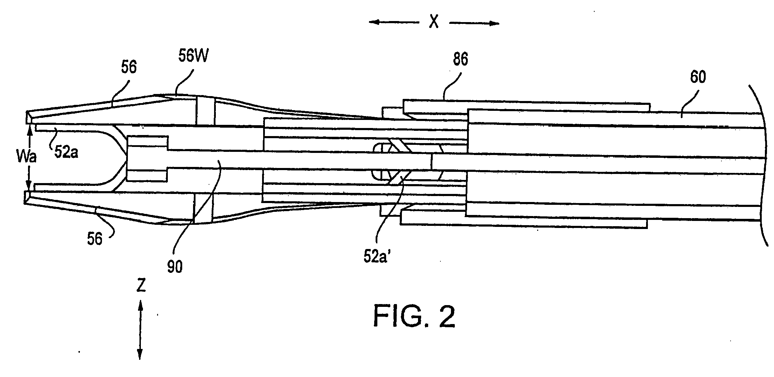

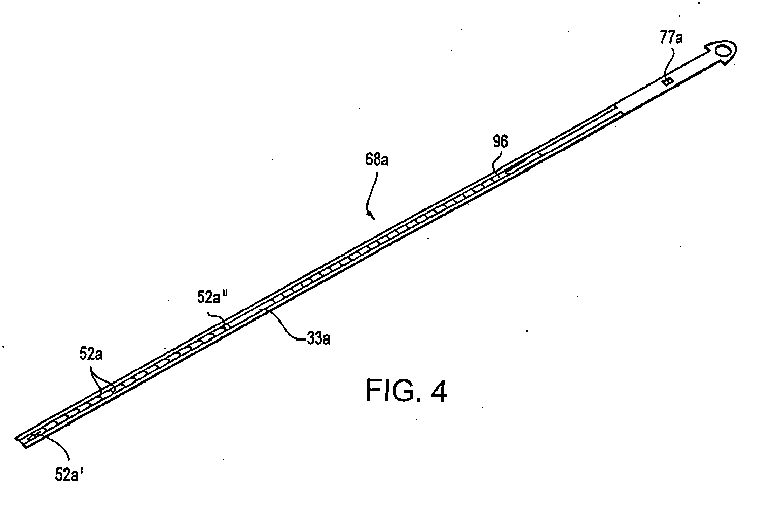

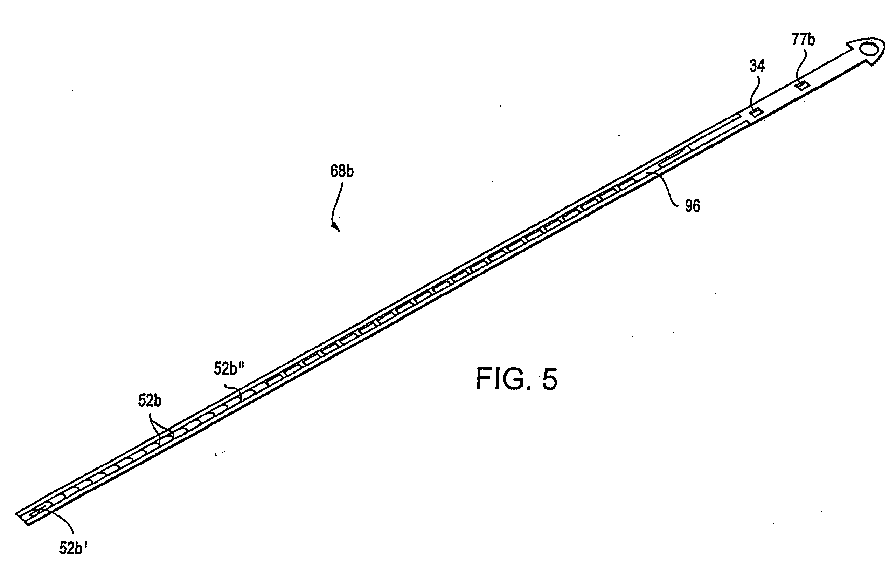

Referring to the drawings, wherein like characters represent like elements, Fig. 1 shows a clip applying device 50 for applying medical tissue-pinching clips 52a, 52b to tissue. The clip applying device 50 has a patient-engaging distalmost end 54 with a pair of squeezable jaw members (jaws) 56 arranged thereon. An elongated channel member or frame (also referred to as the spine) 58 is surrounded by an elongated tube 60, which elongated tube 60 and elongated channel member 58 are secured at their respective proximalmost ends to the distal end of a generally pistol-like handle grip assembly 62. The handle grip assembly 62 includes an arcuately movable squeezable trigger 64 that pivots about pivot shaft 64P. By releasing the squeezed trigger 64 away from a housing portion 66 of the handle grip assembly 62, a clip 52a (shown in Fig. 2), 52b (shown in Fig. 3) is advanced through the distal end of the elongated channel member 58 and into the jaws 56 from an elongated ladder-like clip supply cartridge 68a (shown in Fig. 4), 68b (shown in Fig. 5), disposed through the elongated channel 58. The elongated clip supply cartridge 68a, 68b is fed into a receiving slot or port 70 in the proximal end of the handle grip assembly 62, the receiving slot being in communication with the elongated channel 58.

A rotatable enclosure barrel (also referred to as a tube) 72 is rotatably supported within the handle grip assembly 62. The rotatable enclosure barrel 72 is connected to the proximal end of the elongated channel 58. The enclosure barrel 72 has an annular distal bearing (first bearing) 74 slidably disposed thereon and an annular proximal bearing (second bearing) 76 slidably disposed thereon. While the figures show a cylindrical barrel (*i.e.,* a round cross-section), it should be readily appreciable by those skilled in the art that the barrel may have alternative shapes, including but not limited to oval, square and triangular cross sections. The distal bearing 74 has a compression spring 78 arranged against its distalmost surface 80. The compression spring 78 releases a cinch 86 after the jaws 56 have been closed and a clip 52a, 52b has been crimped, and also provides a proximally-directed bias against the distal bearing 74.

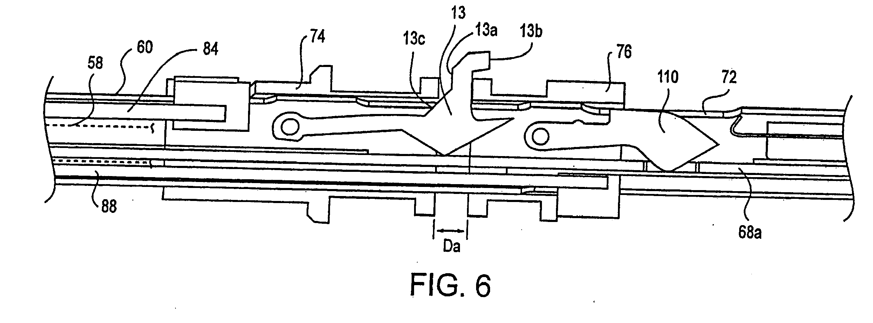

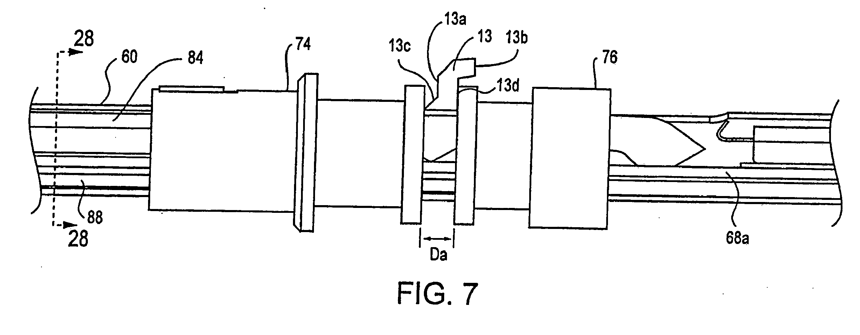

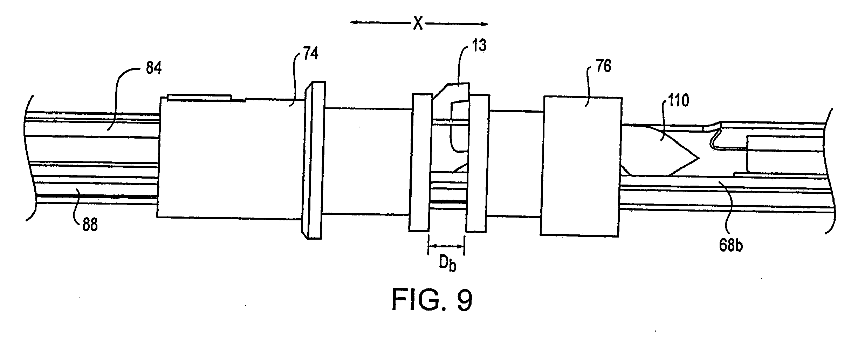

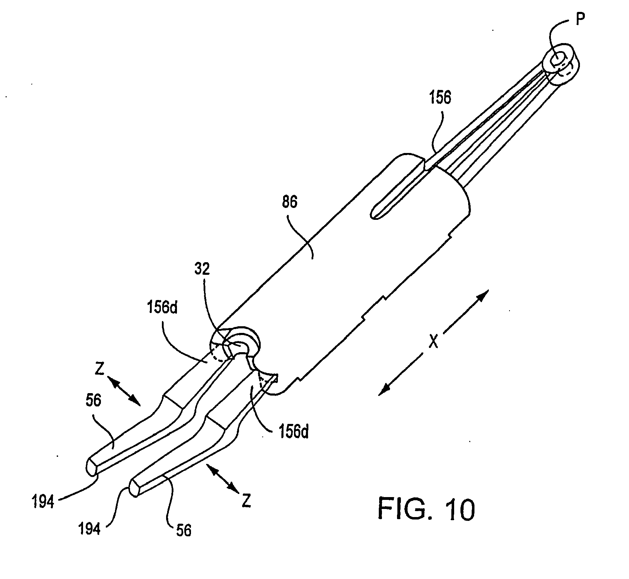

The distal bearing 74 has an elongated cinch rod 84 (shown in Figs. 6-9) extending distally therefrom. The cinch rod 84 extends through the length of the elongated channel 58 (and its surrounding protective enclosure tube 60). The cinch 86 (shown in Fig.10) is generally semi-cylindrically-shaped and arranged on the distal end of the cinch rod 84. The cinch 86 is slidably arranged on the distal end of the elongated channel 58 and is reciprocally slidable in axial direction X to engage the jaws 56 which are squeezably arranged on the distal end of the clip applying device. The each jaw member 56 is located on a respective jaw arm 256, and the jaws 56 and jaw arms are part of a jaw assembly 156. The jaw arms 256 are biased outwardly (*i.e.,* the jaws 56 are biased open). Distal and proximal movement of the cinch 86 in axial direction X and with respect to the elongated channel 58 effects the respective squeezing closure and biased opening of the jaws 56 at the distal end of the elongated channel 58. The cinch rod 84 moves in axial direction X to slide the cinch 86 distally and proximally corresponding to the direction of movement of the distal bearing 74 on the distal end of the enclosure barrel 72.

The distal bearing 74 is biased proximally by the compression spring 78, effecting a proximal motion to the distal bearing 74. The distal bearing 74 is operatively connected to the trigger 64 by a distal paddle 23, and the proximal bearing 76 is operatively connected to a proximal paddle 25, which is in turn connected to the trigger by a trigger linkage 98. Thus, the proximal bearing 76 is biased distally by the compression spring via the trigger linkage 98.

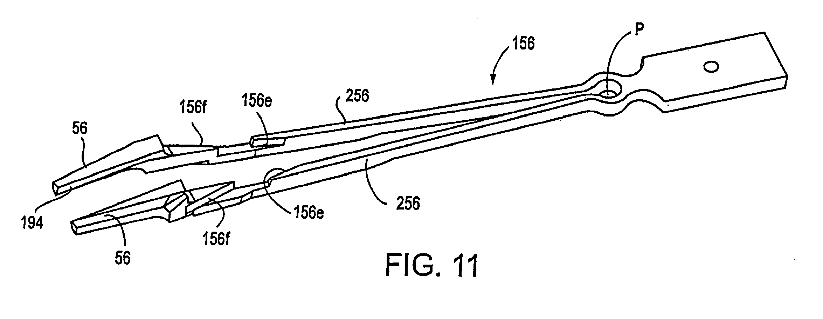

An elongated pusher rod 88 extends adjacent to the lower side of the elongated channel 58 (as shown, *e.g.,* in Figs. 1, 6-9 and 37). The elongated pusher rod 88 has a proximal end connected to the proximal bearing 76 surrounding the enclosure barrel 72 at the proximal end of the handle grip assembly 62. The pusher rod 88 is connected to a clip-engaging feeder 90 (shown in Figs. 2-3). The feeder 90 is movable in axial direction X in relation to the clip-loaded cartridge 68a, 68b disposed within the elongated channel 58; however, the pusher rod is disposed below the elongated channel 58 (shown in Fig. 6). Proximal motion of the feeder 90 is effected by proximal motion of the proximal bearing 76 around the enclosure barrel 72 within the handle grip assembly 62. As the trigger 64 is squeezed (*i.e.*, as the trigger is moved toward the housing portion 66 and pivoted about pivot shaft 64P), the pusher rod 88 distally retracts in the axial direction X due to distal movement of the distal bearing 74, which distally moves feeder 90 to retrieve a distalmost clip 52a', 52b' of the plurality of clips that are serially loaded in respective clip cartridges 68a, 68b, in order to prepare (upon release of the trigger) to load the distalmost clip 52a', 52' into guide slots 194 (shown in Figs. 10-11 and 19), in the opposed faces of the respective jaws 56. The term "plurality" as used throughout the specification is to be interpreted to mean greater than one and within a number understood by those skilled in the art.

The trigger 64 is biased toward the unsqueezed position by the compression spring 78 (shown in Fig. 1). Upon release of the trigger 64, the distal bearing 74 is biased to move proximally, resulting in proximal (rearward) movement of the cinch 86 by the proximal movement of the cinch rod 84, which permits the jaws 56 to bias themselves open, and the feeder 90 (pushed by the pusher rod 88) to push the next available (distalmost) clip 52a', 52b' into the guide slots 194 of the respective jaws 56 as the jaws 56 open fully, as the trigger 64 is permitted to open fully (shown in Fig. 1) from the handle 66 portion of the handle grip assembly 62. Release of the trigger 64 (after the trigger 46 has been initially squeezed towards the handle 66) will automatically advance the next available distalmost clip 52a', 52b' within the cartridge 68a, 68b.

The embodiments disclosed herein are configured to accept more than one type of cartridge (*e.g.*, cartridges having different sizes and/or shapes). It is noted that throughout the specification, the term "size" to describe the different clips and/or the cartridges also includes shape as well. In a non-limiting embodiment, the clip applying device 50 of present invention accepts a clip cartridge 68a containing a series of medium-large sized clips 52a, and can also accept a clip cartridge 68b containing a series of large-sized clips 52b. It should be readily appreciable by those skilled in the art, however, that in alternative embodiments, the present invention may be configured to accept various other types of clip cartridges, including but not limited to small and medium cartridges, medium and medium-large cartridges, and small and large cartridges. Additionally, while the figures show the clip applying device 50 being able to work with two types of cartridges, it should be readily appreciable by those skilled in the art, however, that in alternative embodiments more than two types of cartridges may be accepted.

Referring to Figs. 6-9, when the clip applying device 50 is loaded with a cartridge 68a, 68b, there is a predetermined distance Da, Db between the proximal bearing 76 and distal bearing 74, this distance being determined by a stop key 13 located in the enclosure barrel 72.

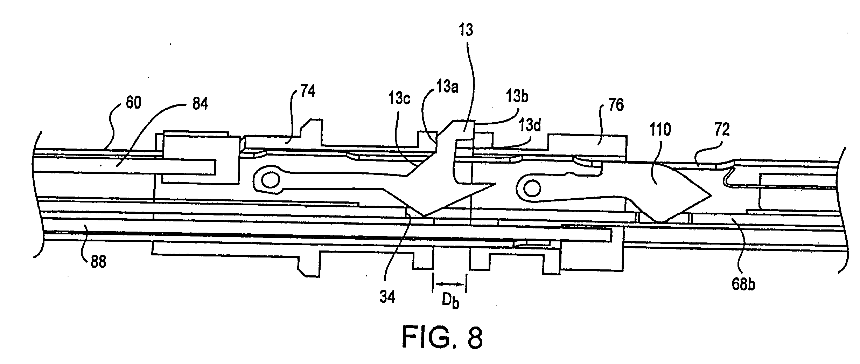

When the clip cartridge 68b is inserted into the receiving slot or port 70 of the clip applying device 50, the cartridge is slid along axial direction X until it reaches a stop (not shown) and the cartridge can no longer be slid. The stop key 13 is then lowered into a stop keyhole 34 (shown in Fig. 5) of the cartridge 68b by pivoting action of the stop key (shown in Figs. 8-9). When the trigger 64 is released (*i.e.,* when the clip applying device 50 is at rest, shown in Fig. 1), the proximal bearing 76 and distal bearing 74 move towards each other such that the stop key 13 is sandwiched therebetween to create a distance Db between the proximal bearing and distal bearing. The distance Db results from the distal bearing 74 contacting a first distal face 13a ofthe stop key 13, and the proximal bearing 76 contacting a first proximal face 13b of the stop key.

When the clip cartridge 68a is inserted by the user into the receiving slot or port 70 of the clip applying device 50, the cartridge slides along axial direction X until it reaches a stop and the cartridge can no longer move axially. The clip cartridge 68a (shown in Fig. 4) does not have a stop keyhole for the lowering of the stop key 13 therein. Thus, when the trigger 64 is released (*i.e.,* when the clip applying device 50 is at rest), the proximal bearing 76 and distal bearing 74 move towards each other such that the stop key 13 is sandwiched therebetween to create a distance Da between the proximal bearing and distal bearing (shown in Figs. 6-7). The distance Da results from the distal bearing 74 contacting a second distal face 13c of the stop key 13, and the proximal bearing 76 contacting a second proximal face 13d of the stop key. The distance Da is less then the distance Db because the proximal bearing 76 and distal bearing 74 are closer to each other in the axial direction X.

Additionally, due to the configuration of the face surfaces 13a-d of the stop key, when the cartridge 68a is inserted into the clip applying device 50, the proximal bearing 76 and distal bearing are both located more distally than when the cartridge 68b is inserted into the clip applying device. Thus (as shown in Fig. 2-3), when the cartridge 68a is inserted into the clip applying device 50, the cinch is advanced in the distal direction to narrow the jaws to a width Wa, and when the cartridge 68b is inserted into the clip applying device 50, the cinch 86 is advanced (a lesser amount than when the cartridge 68a is inserted) in the distal direction to narrow the jaws to a width Wb. The width Wa is narrower than the width Wb because when the cartridge 68a is inserted into the clip applying device, the cinch 86 is distally advanced a greater amount in the axial direction X than when the cartridge 68b is inserted into the clip applying device. As described further hereinbelow, each jaw member 56 is increasingly tapered from a proximal point along the axial direction X to a distalward point where a maximum width portion 56W is located. Thus, the more the cinch 86 is advanced distally toward the maximum width portion 56W, the narrower the width between the jaws. In this way (shown in Fig. 2), the narrower width Wa allows the jaws 56 to securely accommodate medium-large clips 52a in the guide slots 194.

Similarly (shown in Fig. 3), when the cartridge 68b is inserted into the clip applying device 50, since the cinch 86 is advanced a lesser amount than when the cartridge 68a is inserted, the jaws are opened wider to width Wb to allow the jaws 56 to securely accommodate large clips 52b in the guide slots 194, which may have a width larger than smaller clips (*e.g.,* medium-large clips 52a).

Further (as shown in Fig. 2-3), when the cartridge 68a is inserted into the clip applying device 50, the feeder 90 is advanced to the proper position to load the clip 52a, and when the cartridge 68b is inserted into the clip applying device 50, the feeder 90 is advanced (a lesser amount than when the cartridge 68a is inserted) to properly load the larger clip 52b. In other words, when the cartridge 68b is inserted, the feeder 90 does not distally advance as much as when the cartridge 68a is inserted, because the feeder must be further back to accommodate the larger clip 52b (shown in Fig. 3), which may have a longer length than smaller clips (e.g., medium-large clips 52a).

In the above-described embodiment, the position in the axial direction X of both the cinch 86 and the feeder 90 is determined by the stop key 13; however, it is appreciable by those skilled in the art that in alternative embodiments, the position in the axial direction X of only one of the cinch 86 and the feeder 90 may be determined by the stop key, *i.e.,* the stop key may be differently configured to allow varied displacement between the proximal bearing 76 and distal bearing 74. For example, when two clip types of different widths but of the same lengths are respectively used in two different cartridge types, the displacement in the axial direction X of only the cinch may be varied depending on which cartridge type is used, because the feeder is displaced by the same amount in either clip type. Similarly, when two clip types of different lengths but of the same widths are respectively used in two different cartridge types, the displacement in the axial direction X of only the feeder may be varied depending on which cartridge type is used, because the distance between the jaws does not need to be adjusted.

It is noted that in a non-limiting embodiment, either or both cartridges 68a, 68b may contain safety keyholes 77a and 77b (shown in Figs. 4-5) which do not interact with the stop key 13, but rather accept a safety key 110, in order to prevent insertion or withdrawal of the clip cartridge 68a, 68b unless the trigger 64 is generally fully squeezed rearwardly toward the handle 66 of the handle grip assembly 62 in a manner similar to that disclosed in commonly-assigned U.S. Patent Publication No. 2003/0040759.

It is also noted that in a non-limiting embodiment, the trigger 64 may be squeezed to allow the insertion of the cartridge 68a, 68b and/or to crimp a clip 52a, 52b; however, it should be appreciated that in alternative embodiments, other structure may be used (*e.g.,* a separate or integral tab, button, lever or switch), which may be activated to allow the cartridge 68a, 68b to be inserted (and/or to crimp a clip 52a 52b), such that the position in the axial direction X of the proximal bearing 76 or the distal bearing 74 may be determined.

The cinch 86 and jaw assembly 156 of the present invention allow full, positive and consistent control of the opening and closing of the jaws 56 in direction Z (shown in Figs. 2 and 10), hereinafter referred to as radial movement, or movement in the radial direction. Because the clip applying device 50 is able to accept two different types of clip cartridges 68a, 68b, and the jaws 56 thus move radially over a greater distance than other clip appliers, such full, positive and consistent control of the jaws 56 is required. Further, limited travel in the axial direction X of a cinch is normally not enough to cover this increased radial travel of the jaws 56. In other words, there is no single surface of the cinch 86 or jaw assembly 156 to completely and accurately control the opening and closing of the cinch.

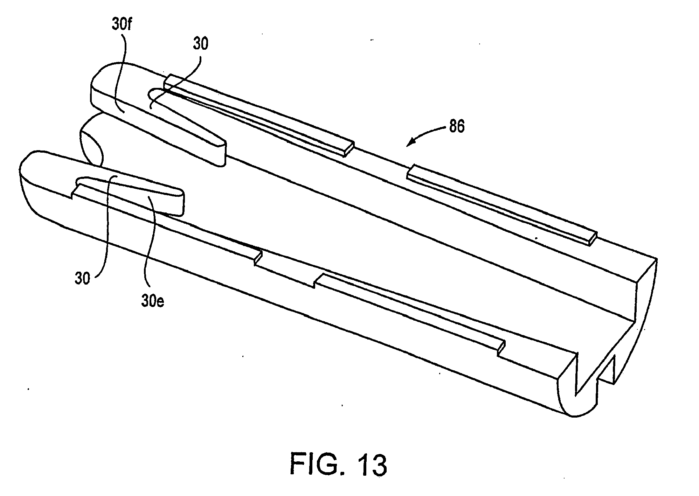

An embodiment of the present invention includes a cinch 86 that allows for greater radial movement of the jaws 56 by engaging different jaw engagement surfaces 156a, 156b, 156c,156d,156e,156f of each jaw arm 256. The generally semi-cyundrically-shaped cinch 86 has a pair of control fingers (or control members) 30 (shown in Fig.13) on the underside thereof which engage inner engagement surfaces 156e and outer engagement surfaces 156f of the jaw arms. In a non-limiting embodiment, the inner and outer engagement surfaces 156e, 156f, as well as the control fingers 30, may be tapered or angled.

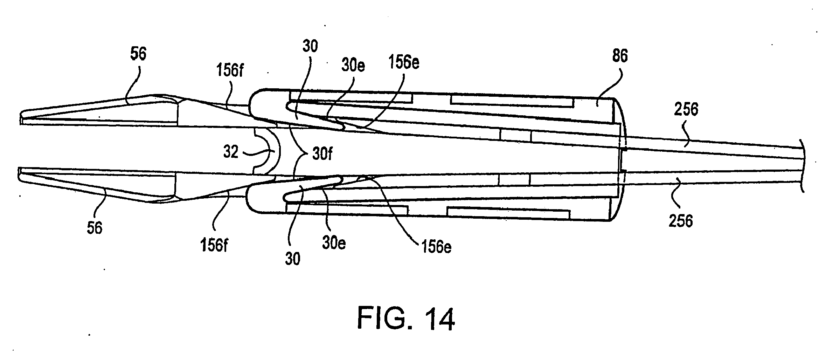

Fig. 14 shows the cinch 86 in an intermediate position, where the control fingers 30 are intermediate the respective inner engagement surfaces 156e and outer engagement surfaces 156f. In this position (in a non-limiting embodiment), medium-large clips 52a may be loaded and the jaw arms may be at rest (*i.e.,* spring forces of the jaw arms are acting neither inwardly nor outwardly in the radial direction Z).

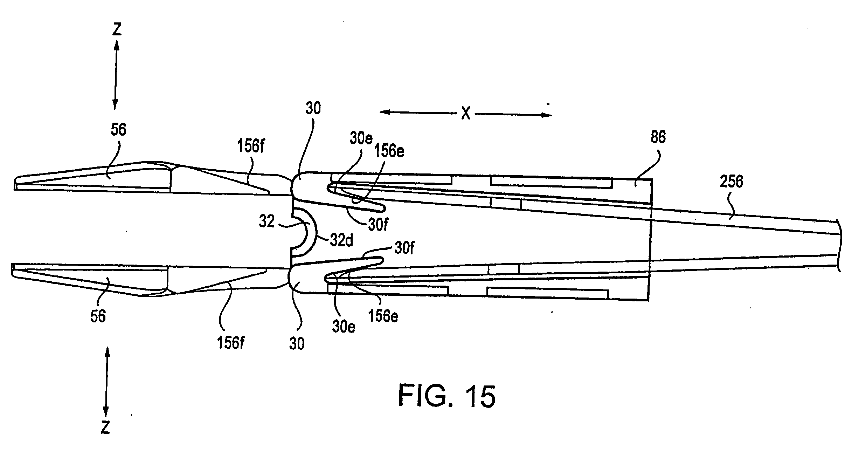

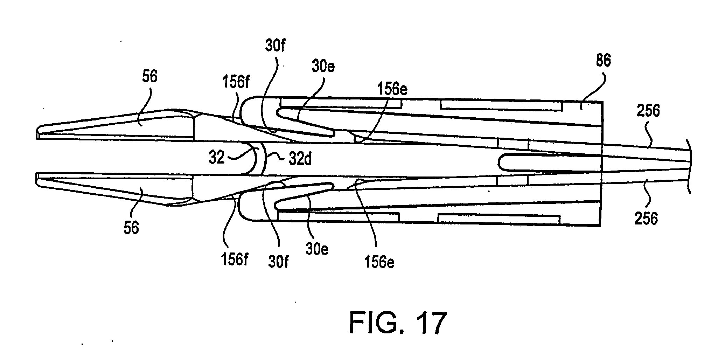

When the cinch is moved proximally in the axial direction X from the intermediate position (*e*.*g*., toward a first position), outer control surfaces 30e of the control fingers 30 respectively engage the inner engagement surfaces 156e to widen the gap between the jaws 56 (*e.g.,* to a width Wb, in order to accommodate cartridge 68b containing large sized clips 52b), while inner control surfaces 30f of the control fingers are brought out of contact with respective outer engagement surfaces 156f, as shown in Figs. 15 and 17. Conversely, when the cinch is moved distally from a proximalmost position toward the intermediate position, the outer control surfaces 30e of the control fingers respectively slide along the inner engagement surfaces 156e to gradually (due to the angled configuration) let the jaw arms 256 bias themselves inward.

Similarly, when the cinch is moved distally in the axial direction X from the intermediate position (e.g., toward a second position), inner control surfaces 30f of the control fingers 3 0 respectively engage the inner engagement surfaces 156e to narrow the gap between the jaws 56 (e.g., to a width Wa, to accommodate cartridge 68a containing medium-large sized clips 52a, or to begin a clip-crimping operation), while outer control surfaces 30e of the control fingers are brought out of contact with respective outer engagement surfaces 156e, as shown in Figs. 15 and 17. In a non-limiting embodiment, in order to ensure smooth transfer of control throughout the range of movement of the cinch 86, the control fingers 30 are preferably always in contact with at least one of the inner or outer engagement surfaces 156e, 156f (as shown in Fig. 14).

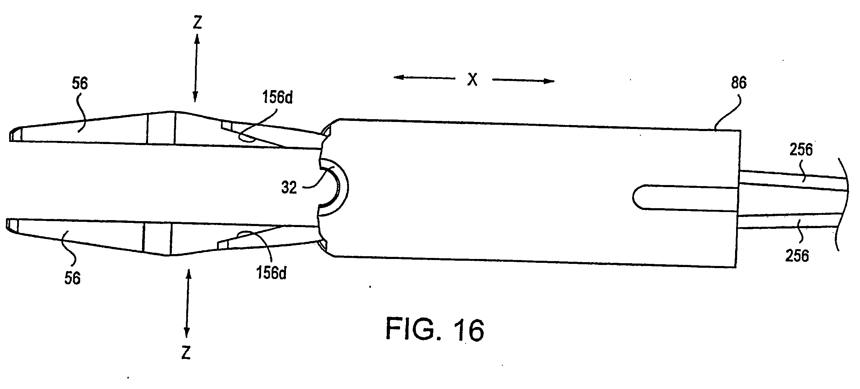

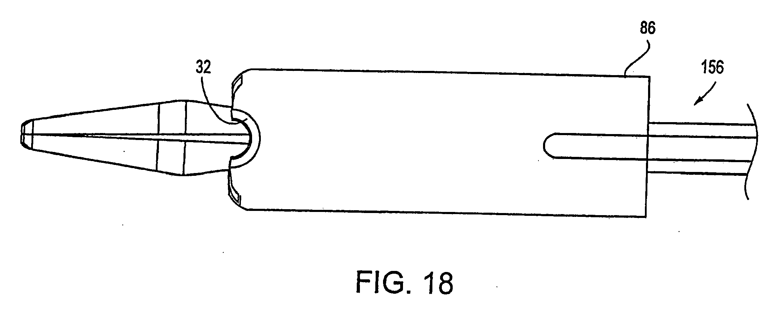

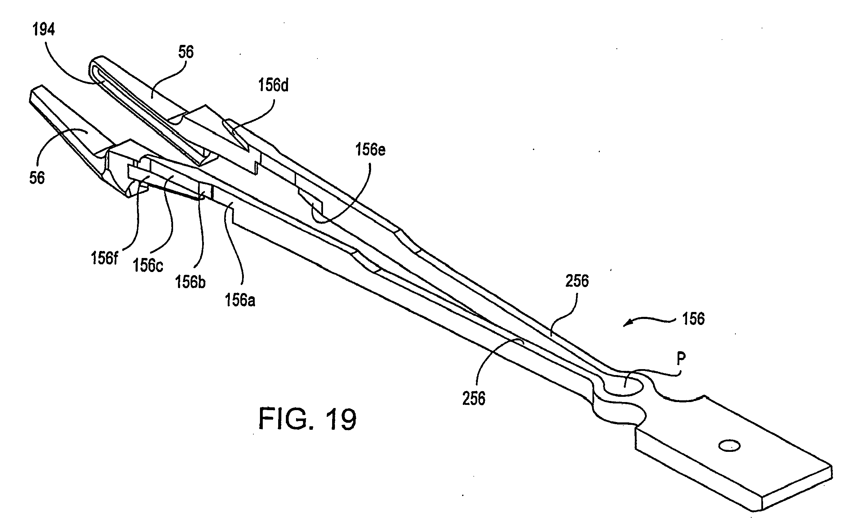

Fig. 18 shows the cinch 86 in a fully distal position in which a clip 52a, 52b may be fully crimped closed. In a non-limiting embodiment, in order to move the cinch 86 from the fully-distal position along the axial direction X, the cinch includes a generally arcuate central block 32 having opposed ends which respectively engage second inner engagement surfaces 156d located on an upper surface of each jaw arm 256 (shown in Figs. 16 and 19), which may be tapered or angled in a non-limiting embodiment. The central block 32 ensures accurate and precise transition of the control surfaces of the cinch 86 when the jaws 56 begin to open (*i.e.* when the cinch moves proximally along axial direction X) from the closed jaw position. For example, when the cinch 86 begins to open from the closed jaw position (shown in Fig. 18), a proximal facing surface 32d of the central block 32 engages the second inner engagement surfaces 156d commence an opening operation of the jaws. After the cinch 86 moves proximally partway along the axial direction X toward the intermediate position, control of the opening of the jaws 56 is "handed off" to the inner control surfaces 30f of the control fingers 30, which respectively slide along the outer engagement surfaces 156f to gradually (due to the angled configuration) provide for outward movement of the jaw arms 256 (in direction Z). Although the central block 32 is shown in the figures as being arcuate, it should be understood by those skilled in the art that the central block may take a variety of shapes, including but not limited to, e.g., a wedge, circle, a V or W shape.

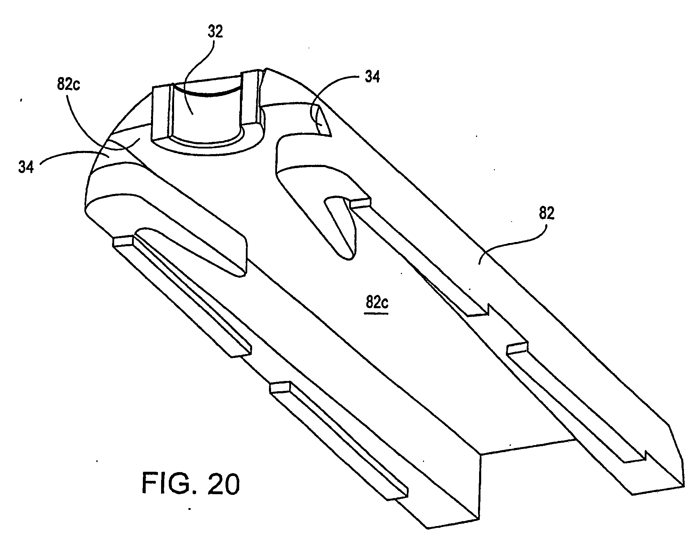

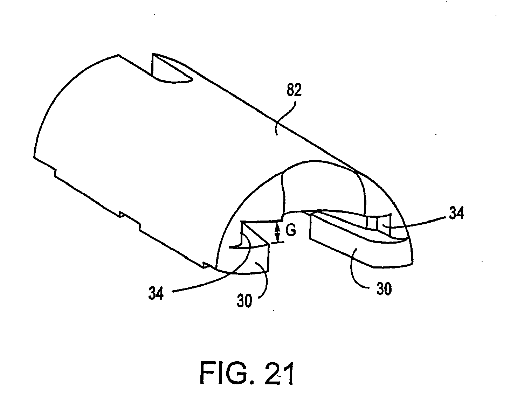

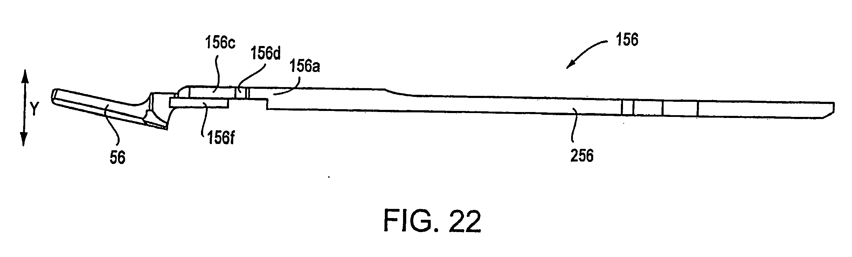

In a non-limiting embodiment, the cinch 82 may include a pair of inwardly facing guide walls 34 (shown in Figs. 20-21) configured to respectively slidingly engage one or more outer side engagement surfaces 156a-c (shown in Figs. 19 and 22) of each jaw arm 256 to provide further control of (e.g., pitch in the radial direction) the jaw assembly 156 by the cinch. With such a configuration it may not be necessary for the jaw assembly 156 to remain at rest in the intermediate position. Additionally, since the control fingers 30 protrude inwardly, a gap or channel G may be present between the control finger 30 and the ceiling 82c of the cinch 82. A portion of each jaw arm 256 may then be sandwiched between a respective control finger 30 and the ceiling 82c of the cinch 82, thereby providing control and stability (by e.g., preventing dive) of the jaws in a Y-axis direction as the cinch slides in the X-axis direction.

In Figs. 2 and 10, the radial opening and closing direction of the jaws 56 appears to be orthogonal to the axial direction X (*i*.*e*., it appears to be parallel to the axial direction Z); however, it should be understood by those skilled in the art that orthogonal radial movement of the jaws is neither necessary nor required. As shown in Fig. 10, in a non-limiting embodiment, the jaw arms 156 may pivot about pivot point P and as such, may result in the radial movement of the jaws being oblique (*i.e.,* in a radial oblique direction) to the axial direction X.

It is also noted that the cinch control arrangement of the present invention is not limited to a medical clip applier device that can accept more than one type of cartridge, or to a clip applier that can hold a single type of cartridge, or even to a clip applying device having clips pre-installed therein. Rather, it should be understood by those skilled in the art that the above-described cinch control arrangement of present invention may be used in other medical instruments aside from clip-applying devices (e.g., shears, graspers or grippers) where a cinch is moved axially to widen and/or narrow a gap between a pair of jaws or other opposed members.

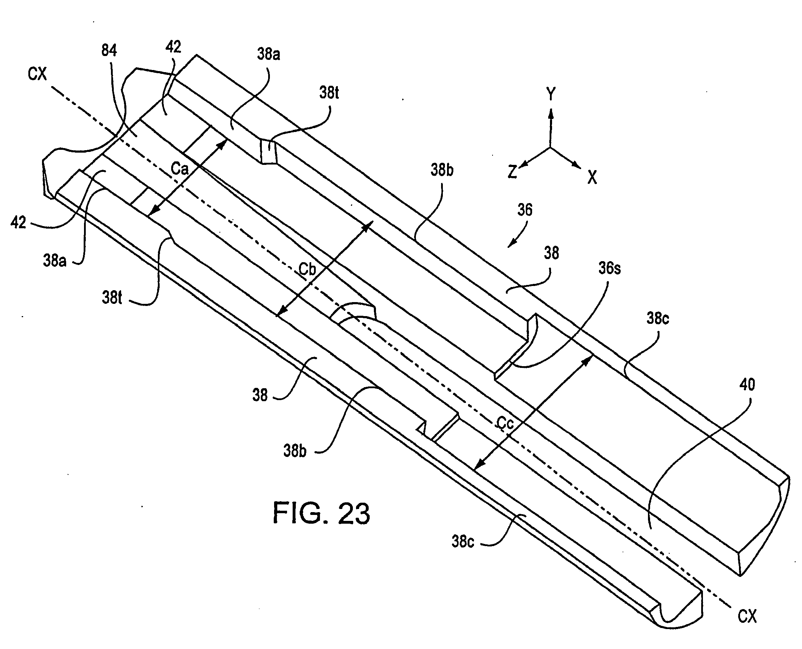

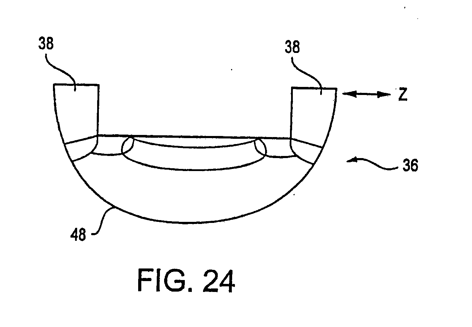

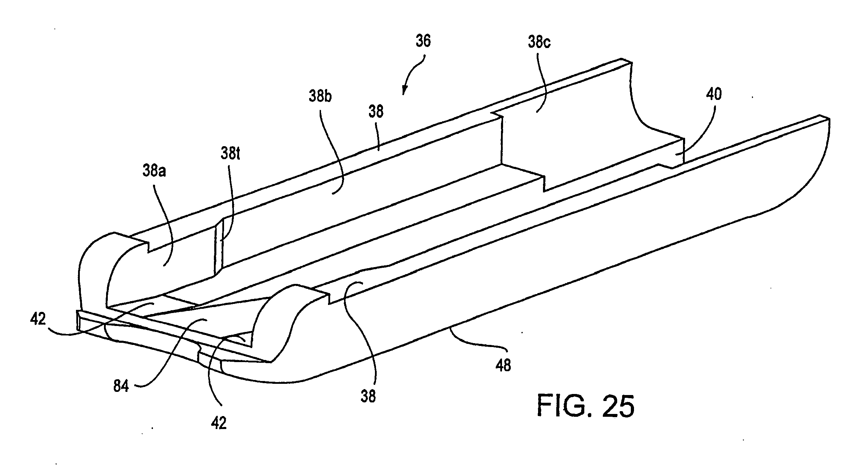

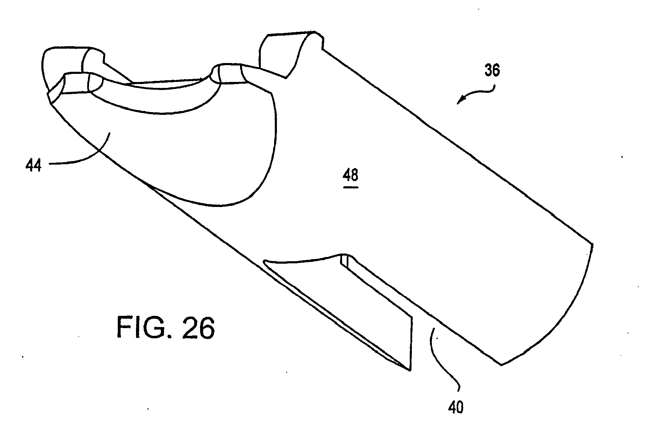

As described above, the clip applying device 50 of the present invention is able to accept at least two different types of clip cartridges 68a, 68b, and as such, the present invention also provides a cartridge alignment device 36 (shown in Figs. 23-26) that can center (along central axis CX) clip cartridges 68a, 68b within the elongated tube 60 in a width, or radial direction Z of the cartridges. In other words, the alignment device 36 can maintain the loaded cartridges 68a and 68b in a central position in relation to the sides (*i.e.,* equidistant to the sidewalls of the tube 60 in the radial orthogonal Z axis direction) of the elongated tube 60 (*i.e.,* so that a sagittal plane defined by the X-Y axis bisecting the cartridges 68a, 68b is substantially coplanar with a sagittal plane X-Y that bisects the elongated tube. With such an arrangement, the clip cartridge 68a, 68b can be centered within the elongated tube and the clip cartridge 68a, 68b and the elongated tube, are aligned in the axial direction X (*i.e.,* along the central axis CX). Thus, a clip 52a, 52b can be accurately loaded in the jaws 56 and accurately crimped thereby.

The alignment device 36 has a pair of elongated rails 38 extending in the axial direction X. The rails 38 each have a distal (first) region 38a defining a first channel Ca therebetween, a middle (second) region 38b defining a second channel Cb therebetween, and a proximal (third) region 38c defining a third channel Cc therebetween. Each channel Ca, Cb and Cc successively extends in communication and in the axial direction X. In a non-limiting embodiment, the distal channel Ca is narrower than the middle channel Cb, which in turn is narrower than the proximal channel Cc. The alignment device also has a pair of transition areas 38t (each located between a respective distal region 38a and middle region 38b) which may be tapered or angled to accurately guide the insertion of cartridge 68a into the distal channel Ca, where the cartridge 68a is securely held and centrally aligned therein in the radial direction Z (*i.e.,* cartridge 68a is sandwiched between both distal regions 38a), due to the extending of the distal channel Ca in the axial direction X. In situations where the cartridge 68b is wider in radial direction Z than the cartridge 68a, the cartridge 68b cannot fit within the distal channel 68a. Rather, the transition area blocks the cartridge 68b from being inserted (in the axial direction X) in the distal channel 68a, and also prevents damage to the cartridge 68b during the insertion process. Thus, the cartridge 68b is securely held and centrally aligned in the channel Cb in the radial direction Z (*i.e.,* the cartridge 68b is sandwiched between both middle regions 3 8b), due to the extension of the middle channel Cb in the axial direction X.

The proximal channel Cc is configured to hold the elongated channel member 58 (through which the cartridge 68a, 68b is inserted), which is inserted therein during the manufacturing/assembly process. Thus, the elongated channel member 58 is also securely held and centrally aligned in the proximal channel Cc in the radial direction Z (*i.e.,* the elongated channel member 58 is sandwiched between both proximal regions 38c), due to the extension of the middle channel Cb in the axial direction X. Additionally, the alignment device 36 also includes a stop region 36s which prevents distal displacement in the axial direction X of the elongated channel member 58. The thickness of a lower portion of the elongated channel member 58 is preferably the same thickness as the lower portion of the stop region, thereby ensuring smooth loading of the cartridges 68a, 68b into their respective channels Ca, Cb.

The alignment device 36 may further include an axially-extending cavity 40 configured to accept the insertion and retraction of the clip-engaging feeder 90 when it loads a clip 52a, 52b (*i.e.,* the cavity 40 can accommodate the feeder 90 therein, between the rails 38), and may further include a ramp portion 42 configured to facilitate the sliding and loading of a clip 52a, 52b into guide slots 194 of the jaws 56. The alignment device may also include a middle inclined region 84, located distally of the cavity 40 and extending in the axial direction X, to assist in the upward distal sliding in the axial direction X of the feeder 90. Further, the alignment device 36 may have an angled or tapered surface 44 at the distal end thereof and/or a semi-cylindrical outer surface 48, to facilitate the insertion of the alignment device, and therefore the clip-applying device 50, into the body cavity.

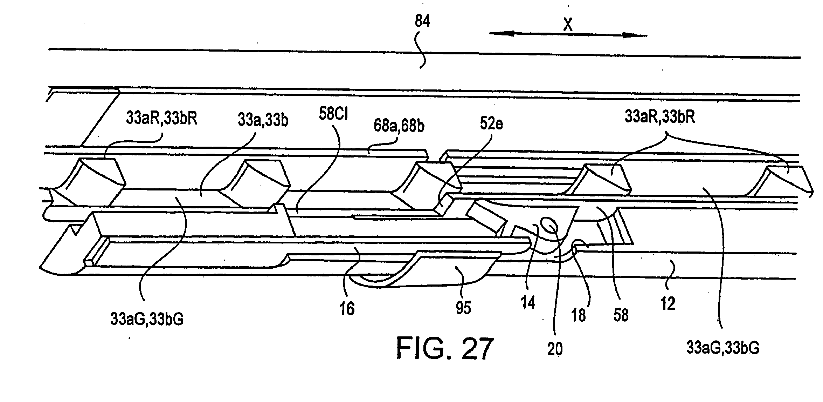

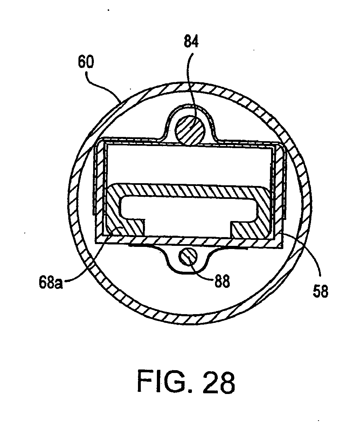

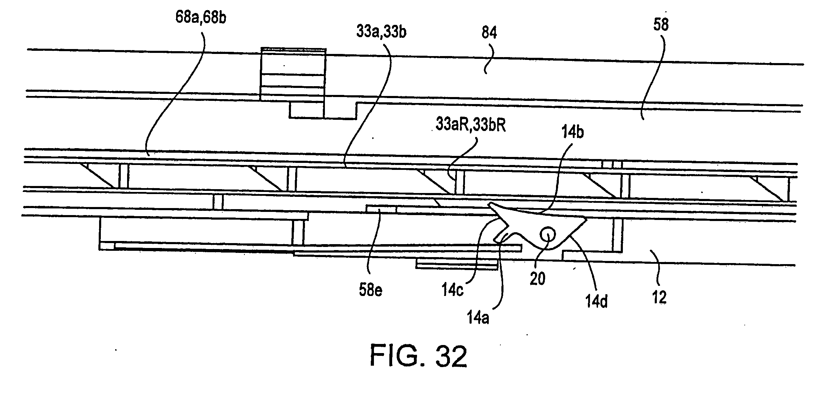

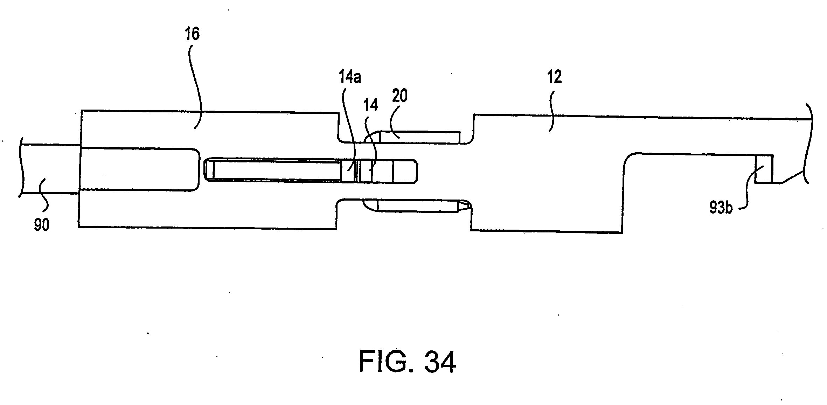

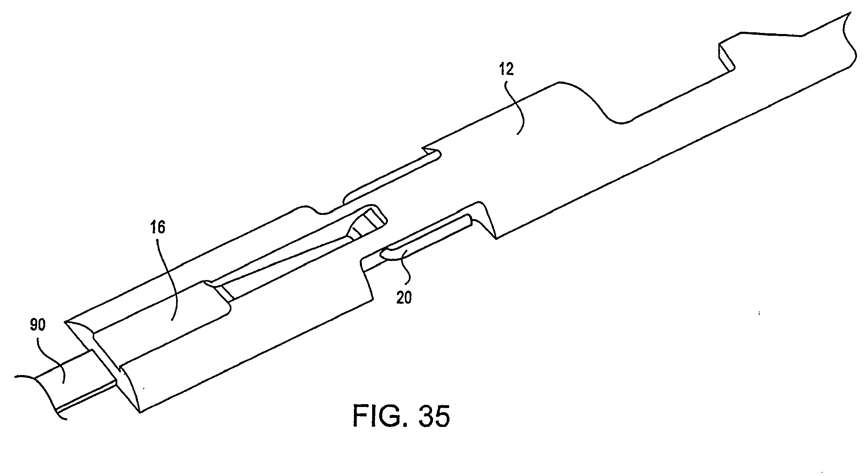

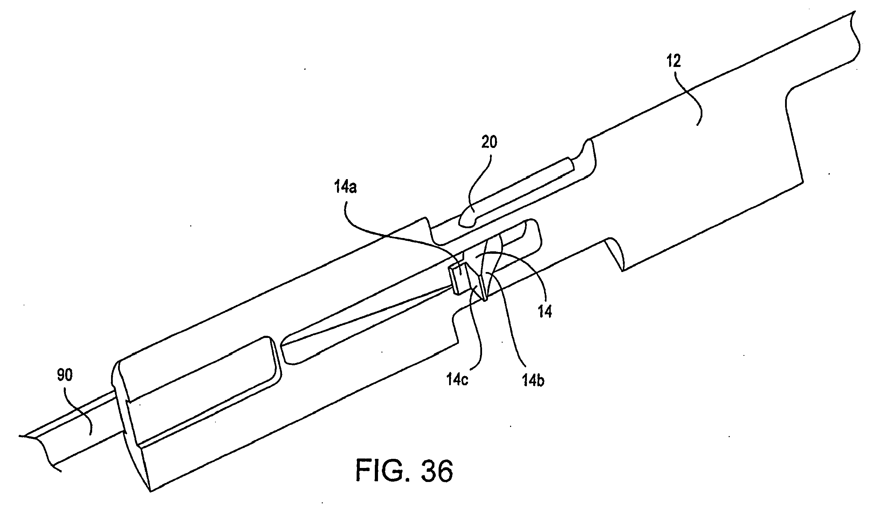

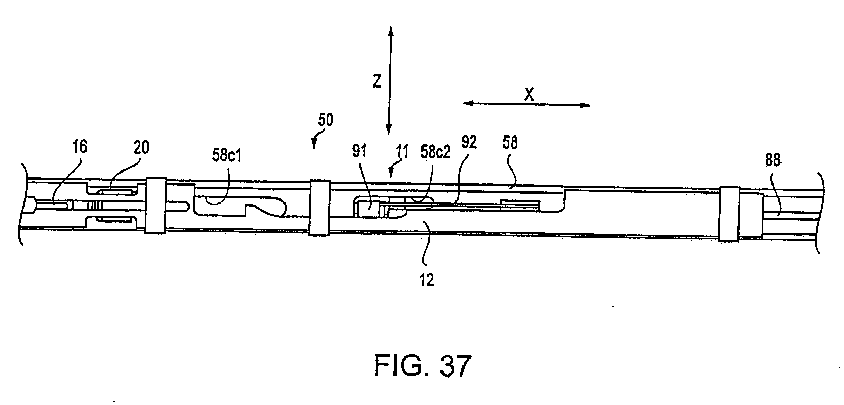

An advancement block (also referred to as a slider) 12 (shown in Figs. 27 and 29-36) is attached between the pusher rod 88 and clip-engaging feeder 90 (shown in Figs. 2-3 and 37). The feeder 90 is movable in axial direction X in relation to the clip-loaded cartridge 68a, 68b disposed within the elongated channel 58; however, the pusher rod is disposed below the elongated channel 58 (shown in Figs. 6 and 28). The slider 12 has a ladder-engaging toggle 14, which is configured to sequentially engage a series of openings in ladder member 33a, 33b (respectively shown in Figs. 4-5) slidably arranged within a respective clip cartridge 68a, 68b. The ladder member 33a, 33b, which is engaged by the toggle 14 of the slider 12 (the slider being operatively connected to pusher rod 88 as shown in Fig. 37) is pushed in the distal direction (along axial direction X) to in turn push against the proximalmost or last clip 52a", 52b" in the cartridge 68a, 68b, so as to also push distally the next adjacent clip(s) 52a, 52b within that cartridge 68a, 68b. Forward (or distal) advance of the series of clips 52a, 52b loaded within the cartridge 68 is thus effected.

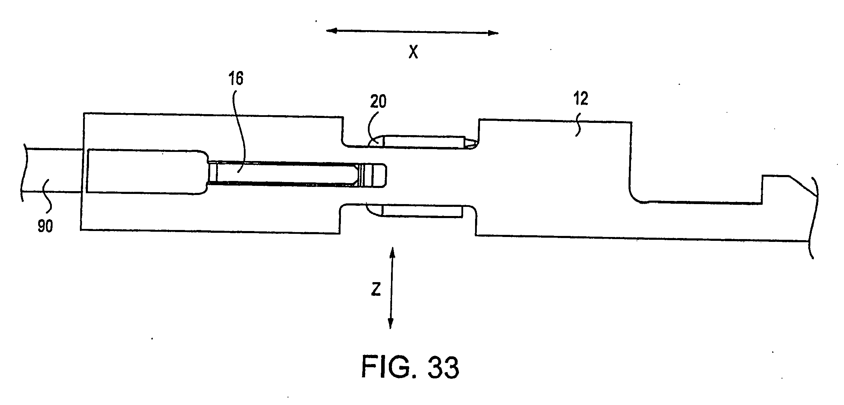

As shown in Figs. 27 and 29-36, the ladder-engaging toggle 14 pivots in both directions about a pivot shaft 20, at least a portion of the pivot shaft generally extending in the orthogonal radial direction Z (*i.e.,* generally orthogonal to the axial direction X). Thus, the toggle is pivotable about the Z axis. As best shown in Figs. 33-36, the pivot shaft 30 may be generally shaped like a U or J, so that the pivot shaft does not slide out of the slider 12. Alternatively, the pivot shaft may have elongated shaft ends, rivets and the like to prevent such sliding out of the slider 12.

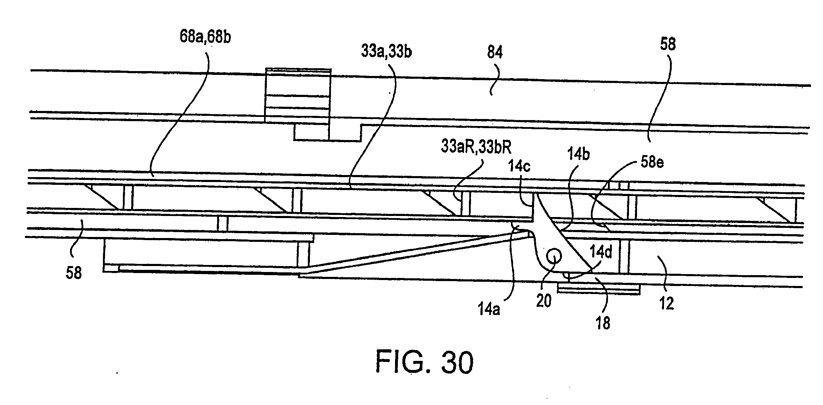

In a non-limiting embodiment, the toggle 14 is generally shaped like a right triangle, but it should be readily appreciable by those skilled in the art that the toggle can take any variety of desirable shapes and configurations. The toggle 14 has a ridge 14a that engages a toggle spring16 affixed to the slider 12. The spring 16 may be a leaf spring (but can be other biasing devices including but not limited to a coil spring or other spring-like members formed, e.g., of an elastomeric material), which engages the ridge 14a to bias the toggle in an upright (second) position, shown in Figs. 30-31 and 36. In a non-limiting embodiment and as shown in Fig. 28, the channel is generally U-shaped, and as described above, the slider 12 proximally and distally slides below and adjacent to the channel 58 in the axial direction X. The channel 58 additionally includes a toggle aperture 58C1 in the bottom portion thereof, through which a portion of the toggle 14 is inserted, when the toggle is in the upright position.

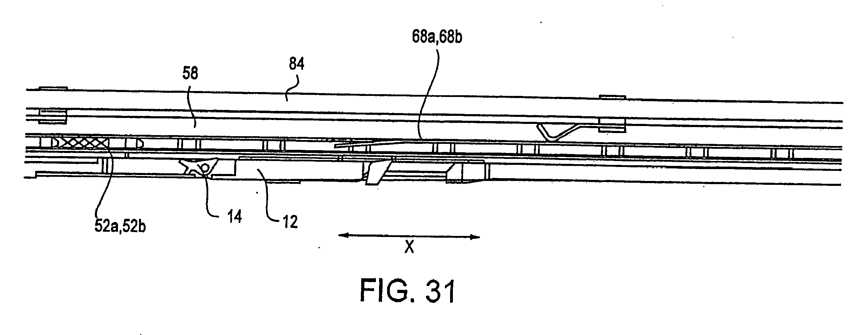

Operation of the clip advancement arrangement according to an embodiment of the present invention will now be described. When the trigger 64 is squeezed, the slider 12 is moved proximally in the axial direction X (since the slider is operably connected to the pusher rod 88, which is in turn connected to the proximal bearing 76). As the slider 12 is moved proximally, an edge-engaging face 14b of the toggle 14 engages an edge 58e of the channel 58, such that the channel edge 58e (which generally extends in the Z axis direction) pushes the toggle 14 to rotate in a first direction (*e.g.,* counterclockwise when viewing Figs. 31-32) to a lowered (first) position and against the biasing force of the toggle spring 16, shown in Figs. 31-32. In the first position, the toggle 14 slides in the axial direction X and below the channel 58, with at least a portion of the edge-engaging face 14b contacting the underside of the channel 58. It is noted that the channel edge 58e may be angled or tapered to increase the surface area thereof, thereby ensuring smooth and precise pivoting action of the toggle 14.

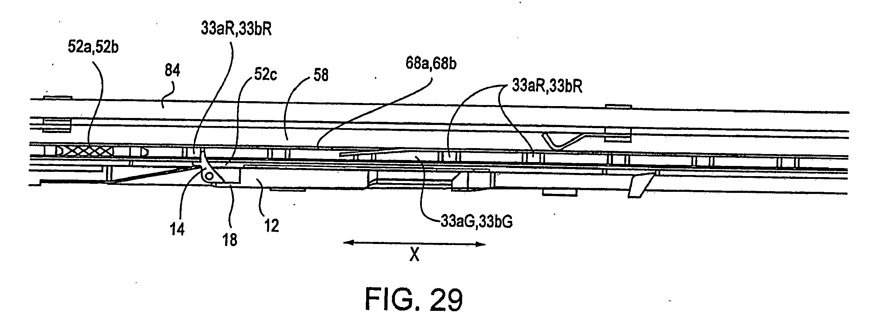

When the trigger 64 is released, the slider 12 begins to distally move in the axial direction X. The edge-engaging face 14b contacts the underside of the channel 58 until the toggle 14 reaches the toggle aperture 58C1, at which point the edge-engaging face 14b contacts the channel edge 58e and allows the toggle spring 16 to urge the toggle into the upright position (shown in Figs. 30-31 and 36) and between rungs 33aR, 33bR of the ladder member 33a, 33b, the rungs having rung gaps 33aG, 33bG therebetween. Thus, the toggle 14 rotates in a second direction (clockwise when viewing Figs. 29-30). Pivoting of the toggle 14 in the second direction is stopped when a heel portion 14d engages a stop portion 18 located on the slider 12.

Once in the upright position, the toggle 14 continues to move distally through the toggle aperture 58C1 (and within a rung gap 33aG, 33bG) in the axial direction X such that a pushing face 14c contacts a rear (proximal) side of a rung 33aR, 33bR of the ladder member 33a, 33b. Thus, the toggle 14 pushes the ladder member 33a, 33b in the distal direction (along axial direction X) to in turn push against the proximalmost or last clip 52a", 52b" in the cartridge 68a, 68b to also push distally the next adjacent clip(s) 52a, 52b. It is noted that while the rungs 33aR, 33bR of the figures extend in the Z axis direction, those skilled in the art will readily appreciate that the rungs could be differently configured in alternative embodiments, including but not limited to, *e.g.,* angled and arcuate.

The above process may be repeated by continually squeezing and releasing the trigger 64, which in turn continually distally advances the ladder member 33a, 33b to distally push clips 52a and 52b, until the last clip 52a", 52b" is loaded into and applied by the jaws 56.

It is noted that while many of the figures show the clip advancement arrangement of the present invention being used in a clip applying device 50 that can accept more than one type of clip supply cartridge 68a, 68b, it should be readily understood by those skilled in the art that the clip advancement arrangement of present invention may be used in a clip applying device that can accept only a single type of cartridge, or alternatively, may be used in a clip applying device having clips pre-installed therein.

Fig. 37 shows a trigger lockout arrangement 11. As described above, the slider 12 is attached between the pusher rod 88 and clip-engaging feeder 90. The trigger lockout arrangement 11 serves to lock movement of the trigger 64 when the last clip 52a", 52b" of the cartridge 68a, 68b has been applied by the jaws 56, thereby reducing the likelihood of tissue damage by squeezing jaws without a staple therein, and alerting the surgeon that additional clips are required.

Also as discussed above, when the trigger 64 is squeezed, the slider 12 proximally moves in the axial direction X, and when the trigger is released, the slider moves in the axial direction X such that the toggle 14 distally advances the ladder member 33a, 33b by one clip and such that the feeder 90 loads the distalmost clip 52a', 52b' between the jaws 56.

The channel member 58 also includes a block 91 affixed to the underside of the channel member via a flexible rod 92, and additionally has a block aperture 58C2, located above the block 91 in the Y-axis direction, for accepting insertion of at least a portion of the block 91 therein. At rest, the flexible rod 92 is biased to generally extend in the axial direction X. The slider 12 also has a tooth 93 that contacts the block 91 when the slider is proximally or distally moved in the axial direction X. While the block 91 is shown as being cubical in arrangement, it should be understood by those skilled in the art that in alternative embodiments the block can take a variety of shapes, including but not limited to, *e.g.,* a trapezoid, frustum or ovoid.

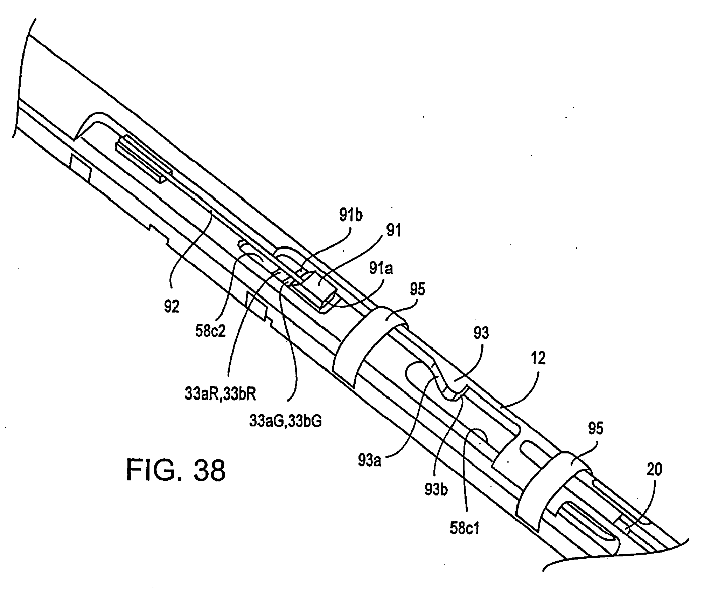

Specifically, as shown in Fig. 38, when the trigger 64 is squeezed and the slider 12 moves proximally in the axial direction X (to approach the block 91 from the front, shown in Figs. 37-38), a rear surface 93a of the tooth 93 engages a front surface 91a of the block 91 to move the block in radial direction Z while the slider continues to move proximally. The rear surface 93a of the tooth 93 is angled to facilitate movement of the block 91 in the radial direction Z. Once the slider 12 passes the block 91, the block returns to its original rest position due to the biasing action of the rod 92.

When the trigger 64 is released, the slider 12 begins to move distally in the axial direction X and approaches the block 91 from the rear (shown in Fig. 39). If a cartridge 68a, 68b having a plurality of clips 52a, 52b has been loaded into the clip applier 50, the slider moves distally such that a front surface 93b (best shown in Fig. 38) of the tooth 93 engages a rear surface 91b of the block 91 to push the block upward in the Y-axis direction (*i.e.,* into the page of Fig. 37). Specifically, the upward movement of the block 91 causes a portion of the block to pass through the block aperture 58C2 of the channel 58 and be inserted between the rungs 33aR, 33bR of the ladder member 33a, 33b (*i.e.,* the portion of the block is inserted into a rung gap 33aG, 33bG), thereby creating clearance for the tooth 93 to pass over the block 91 as the slider continues to move in the distal direction. Once the tooth 93 has passed over the block 91, the rod 92 urges the block downwardly, since the rod 92 is biased in the axial direction X and to keep the block out of the ladder gap 33aG, 33bG.

To facilitate precise movement of the block 91 in the Y-axis direction, the front surface 93b of the tooth 93 and/or the rear surface 91b of the block 91 may be angled. Additionally, one or more bands 95 to slidably secure the slider 12 against the underside of the channel 58 may be provided, thereby securing the slider against movement in the Y-axis direction, when the slider moves in the axial direction X. The presence of the block 91 between the rungs 33aR, 33bR of the ladder member 33a, 33b does not interfere with the distal movement of the ladder member by the toggle, since these two operations are out of phase, *i.e.,* during distal movement of the slider 12, the toggle 14 does not engage a rung 33aR, 33bR until after the tooth 93 has passed over the block 91 and the block has withdrawn from the ladder gap 33aG, 33bG.

As described above, the slider may be continually moved proximally and distally by respective continual squeezing and releasing of the trigger 64, until the last clip 52a", 52b" is loaded into and applied by the jaws 56. While the last clip 52a", 52b" is being loaded into the jaws 56 by the feeder 90 (and thus no more clips 52a, 52b are serially loaded within the cartridge 68a, 68b), the toggle 14 engages the rung 33aR, 33bR, thereby advancing the ladder member 33a, 33b a final time and exposing a solid surface 96 of the ladder member beneath the block aperture 58C2 (*i.e.,* there is no longer a ladder gap 33aG, 33bG beneath the block 91). The clip applying device 50 is now ready for one final clip crimping process. During the final clip crimping process, the trigger 64 is squeezed to proximally move the slider 12 (and to thereby bring the tooth 91 thereof behind the block, as described above) and to distally move the cinch 86 to crimp the last clip 52a", 52b".

As shown in Fig. 40, when the trigger 64 is released after the last clip 52a", 52b" has been crimped, the compression spring 78 urges the trigger toward the open position, which (as described above) moves the slider 12 distally so that the tooth 93 engages the block 91. However, the solid surface 96 of the ladder member 33a, 33b prevents displacement of the block 91 by the tooth 93 in the Y-axis direction (since there is no ladder gap 33aG, 33bG for the block to enter. Thus, the tooth 93 abuts against the block and is prevented from distally moving in the axial direction X, thereby locking the trigger in the closed position and alerting the surgeon that the last clip 52a", 52b" has been applied. As noted above, the bands 95 prevent the slider (and thereby the tooth 93) from being displaced in the Y-axis direction so that the tooth does not "leap over" the block 91, when the slider moves in the axial direction X. It is noted, however, that the trigger 64 is permitted to be slightly unsqueezed so that the last clip 52a", 52b" and any tissue can be released from the jaws 56. Once the cartridge 68a, 68b has been removed from the clip applying device 50, the trigger 64 is free to again move to the unsqueezed position, since the solid surface 96 is no longer present.

An embodiment of the present invention uses cartridges 68a, 68b having twenty clips 52a, 52b, however, it should be understood by those skilled in the art that the present invention may use cartridges having fewer or greater than twenty clips. Additionally, while an embodiment of the trigger lockout arrangement 11 of the present invention locks the trigger 64 after all of the clips 52a, 52b have been applied, it should be understood by those skilled in the art that the trigger lockout arrangement may lock the trigger when one or more clips 52a, 52b remains in the cartridge 68a, 68b or the clip applying device 50.

It is noted that while many of the figures show the trigger lockout arrangement of the present invention being used in a clip applying device 50 that can accept more than one type of clip supply cartridge 68a, 68b, it should be readily understood by those skilled in the art that the trigger lockout arrangement of present invention may be used in a clip applying device that can accept only a single type of cartridge, or alternatively, may be used in a clip applying device having clips pre-installed therein.

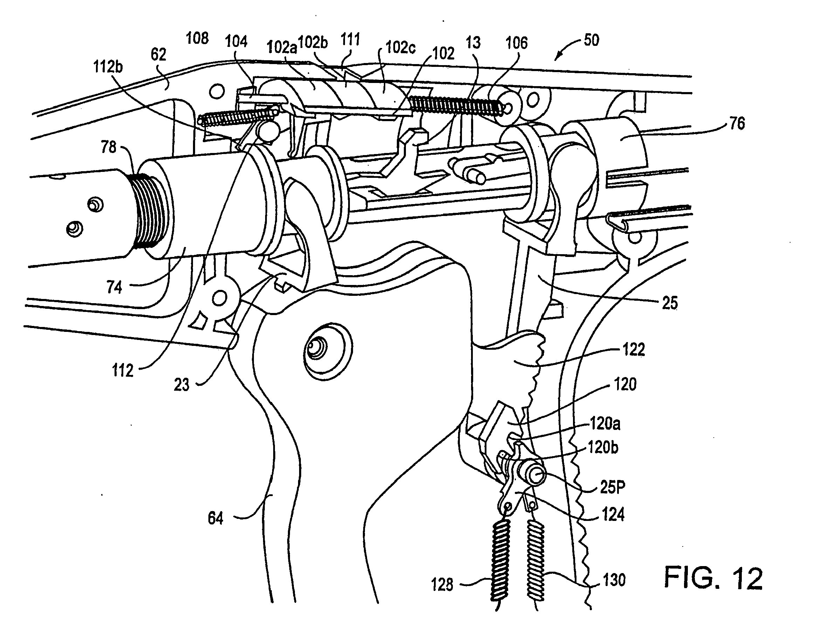

Another feature of an embodiment of the present invention includes a system for indicating to the surgeon when the clip 52a, 52b has been partially crimped (shown in Fig. 42) and/or when the clip 52a, 52b has been generally fully crimped (shown in Fig. 43). Fig 41 shows an uncrimped clip. During surgery, a surgeon may want to apply a clip 52a, 52b, yet still leave a gap between the legs of the clip to allow passage of a blood vessel therethrough, thereby allowing blood to flow through the vessel unobstructed. When a surgeon partially squeezes the trigger 64, the jaws 56 are only partially closed and the clip 52a, 52b may thus be applied in a partially crimped configuration. This partially-squeezed position is referred to as the cholangio zone. It is thus desirable to alert the surgeon (visually, audibly and/or tactilely) as to when the clip applying device 50 is in the cholangio zone (shown in Figs. 12 and 44). It is further desirable to alert the surgeon (visually, audibly and/or tactilely) as to when the clip applying device is in fully squeezed position (shown in Fig. 45).

The clip applying device 50 may include a visual slider 102 (housed within the handle grip assembly 62) which slides along an indicator path by way of indicator shaft 104 in an inclined direction with respect to the longitudinal axis X, although it should be understood by those skilled in the art that the indicator shaft 104 may be parallel or declined with respect to longitudinal axis X in alternative embodiments. The visual slider is biased in the general distal direction by a shaft spring 106 which may be disposed about the proximal end of the indicator shaft. Alternatively, the shaft spring 106 may be disposed at the distal end of the indicator shaft 104 to "pull" the visual slider toward the distal end of the grip assembly 62.

The visual slider 102 includes three indicator regions 102a, 102b, 102c, each having different indicia for indicating the position of the trigger, for example, the color, pattern or wording on each region may be different. Each region 102a, 102b, 102c is alternatively visible through a window 111 located in the top of the grip assembly. As a non limiting example, indicator region 102a (indicating the open trigger position) may be green, indicator region 102b (indicating the cholangio zone) may be yellow, and indicator region 102c (indicating the generally fully-squeezed position) may be red. Alternatively, each indicator region 102a, 102b, 102c may show, e.g., different wording (e.g., "open," "partial," "closed''), a different symbol, or a different pattern.

In the unsqueezed position (shown in Fig. 1), a first abutment surface 102d of the visual slider 102 abuts against a first end 112a of a pivotable trip switch 112 to maintain the visual slider in its distalmost position (against the distal biasing force of the shaft spring 106), and indicator region 102a is displayed through the window 110. The trip switch 112 has a trip switch spring 114 which is affixed to the grip assembly 62 to bias the trip switch to pivot in the counterclockwise direction (when viewed in Fig. 1). Additionally, a second end 112b of the trip switch 112 engages an outer surface of the distal bearing to prevent further counterclockwise pivoting of the trip switch.

When the trigger 64 is squeezed toward the cholangio zone (and the distal bearing begins to move distally along axial direction X), the visual slider 102 remains in position (due to the continued engagement of the first abutment surface 102d with the first end 112a of the trip switch 112) until an angled distal surface of a distal bearing ring 108 engages the second end 112b of the trip switch to pivot the trip switch clockwise (when viewed in Fig. 1), which disengages the first abutment surface 102d from the first end 112a of the trip switch 112, thereby allowing the shaft spring 106 to proximally urge the visual slider 102. The visual slider is proximally urged until the first end 112a of the trip switch 112 engages a second abutment surface 102d of the visual slider 102 to stop proximal movement of the visual slider and exposing the indicator region 102b through the window 111 (indicating the cholangio zone to the surgeon), as shown in Figs. 12 and 44.

When the trigger is squeezed from the cholangio zone to the generally fully squeezed position (and thus the distal bearing continues to move distally to cause the jaws 56 to generally fully crimp the clip 52a, 52b), the outer circumference of the distal bearing ring 108 further engages the second end 112b of the trip switch 112 to further pivot the trip switch clockwise, which disengages the second abutment surface 102d from the first end 112a of the trip switch 112, thereby allowing the shaft spring 106 to further proximally urge the visual slider 102 until the slider reaches its limit of proximal travel along the indicator shaft 104, thereby exposing the indicator region102c through the window 111 (indicating the generally fully squeezed position to the surgeon), as shown in Fig. 45.

When the trigger 64 is released (and the distal bearing begins to move proximally due to the biasing force of the compression spring 78), a proximal face of the distal bearing ring 108 engages an indicator finger 102d to proximally move the visual slider 102 so that the first abutment surface 102d of the visual slider 102 again abuts against a first end 112a of the trip switch 112 to reset the visual slider in its distalmost position (thereby again exposing the indicator region 102a through the window 111), as shown in Fig. 1.

It is noted that, in alternative embodiments, one skilled in the art would readily appreciate that the present invention may be provided with only one indicator region 120a, 120b, 120c, only two indicator regions 120a, 120b, 120c (rather then three as shown in the figures), or more than three indicator regions, to indicate any combination of (a) that the clip-applying device 50 is unsqueezed, (b) that the clip applying device is in the cholangio or other mid-squeeze zone, and (c) that the clip applying device is generally fully squeezed.

The clip applying device 50 of the present invention may also include an audible and/or tactile indicating arrangement. Specifically, in the event the surgeon is too focused on the surgery at hand to look at the viewing window 111, the indicating arrangement may audibly and/or tactilely indicate to the surgeon when the clip applying device is in the cholangio zone and/or when the trigger is generally fully squeezed. Additionally, the tactile indicating arrangement of the present invention may assist the surgeon in the event that the surgeon is hearing-impaired.

As shown in Figs. 12 and 45, an upper (first) ratchet 120 and a lower (second) ratchet 122 are affixed to and move with the trigger 64, which pivots about the trigger pivot shaft 64P. Additionally, an upper (first) pawl 124 and a lower (second) pawl 126 are pivotably affixed about a proximal paddle shaft 25P, about which the proximal paddle 25 is also affixed. Additionally, the upper pawl 124 is biased to pivot counterclockwise (when viewed in Fig. 12) by an upper spring 128, and lower pawl 126 is biased to pivot counterclockwise by a lower spring 130, the upper spring having a stronger spring force than the lower spring.

When the trigger is squeezed from the unsqueezed position toward the cholangio zone, lower spring 130 biases the lower pawl 126 to engage the lower ratchet 122 to create a ratcheting sound as the trigger is squeezed toward the cholangio zone and the upper and lower ratchets pivot counterclockwise about trigger pivot 64P (when viewed in Fig. 12), while indicator region 102a is displayed in the window 111. Once the cholangio zone is entered (and at substantially the same time the window 111 displays indicator region 102b), the upper ratchet 120 and lower ratchet 122 continue to pivot counterclockwise so that the upper ratchet engages the upper pawl 124. Specifically, when the cholangio zone is entered, the upper pawl 124 (due to the biasing force of the upper spring 128) engages the first tooth of the upper ratchet 120 to enter a first channel (cholangio channel) 120a of the upper ratchet to emit a distinct sound and transmit a distinct tactile sensation. The sound and feel of the upper pawl 124 entering the first channel 120a is significantly different than the sound and feel of ratcheting of the lower pawl 126 ratcheting with the lower ratchet 122, due to the spring force of the upper spring being greater than that of the lower spring. Additionally or alternatively, differing the dimensions and/or materials between the upper ratchet 120 and the lower ratchet 122 and/or differing the dimensions and/or materials between the upper pawl 124 and the lower pawl 126 may contribute to the differing sounds and feels when the trigger 64 is squeezed.

When the trigger 64 moves from the cholangio zone to the generally fully squeezed position (and at substantially the same time the window 111 displays indicator region 102c), the upper ratchet 120 and lower ratchet 122 continue to pivot counterclockwise such that the upper pawl moves from the cholangio channel 120a to a generally fully squeezed channel 120b, to emit the distinct sound and transmit the distinct tactile sensation. It is noted that the two distinct sounds and sensations (*i*.*e.*, of the upper pawl 124 engaging the upper ratchet 120) can be made different by differing the dimensions and/or material of the cholangio channel 120a and the generally fully squeezed channel 120b. It is further noted that in alternative embodiments, the generally fully squeezed channel 120b may be eliminated, thereby alerting the surgeon only to when the clip applying device 50 is in the cholangio zone.

When the trigger 64 is released, the trigger moves to the unsqueezed position to disengage the upper pawl 124 from the upper ratchet 120, thereby resetting the clip applying device 50. The upper ratchet 120/upper pawl 124 and the lower ratchet 122/lower pawl 126 lie in different X-Y planes (different sagittal planes) so that the upper ratchet does not ever engage the lower pawl and so that the lower ratchet does not ever engage the upper pawl.

It is noted that while many of the figures show the indicating arrangement of the present invention being used in a clip applying device 50 that can accept more than one type of clip supply cartridge 68a, 68b, it should be readily understood by those skilled in the art that the indicating arrangement of present invention may be used in a clip applying device that can accept only a single type of cartridge, or alternatively, may be used in a clip applying device having clips pre-installed therein.

It is further noted that the foregoing examples have been provided merely for the purpose of explanation and are in no way to be construed as limiting of the present invention. While the present invention has been described with reference to a preferred embodiment, it is understood that the words which have been used herein are words of description and illustration, rather than words of limitation. Changes may be made, within the purview of the appended claims, as presently stated and as amended, without departing from the scope and spirit of the present invention in its aspects. Although the present invention has been described herein with reference to particular means, materials and embodiments, the present invention is not intended to be limited to the particulars disclosed herein; rather, the present invention extends to all functionally equivalent structures, methods and uses, such as are within the scope of the appended claims.

## Claims

1. A mechanism for opening and closing jaws of a medical instrument, the mechanism comprising:
a cinch configured to selectively move in opposite directions along a longitudinal axis, said cinch comprising a pair of control members each having an inner control surface and an outer control surface; and
a jaw assembly having a pair of jaw arms, eachjaw arm comprising an inner engagement surface configured to slidingly engage with a respective one of said outer control surfaces, and an outer engagement surface configured to slidingly engage with a respective one of said inner control surfaces.

2. The mechanism according to claim 1, wherein when said cinch moves rearwardly from an intermediate position, said inner engagement surfaces slidingly engage with respective said outer control surfaces to move said pair of jaw arms apart.

3. The mechanism according to claim 1, wherein when said cinch moves forwardly from an intermediate position toward a closed jaw position, said outer engagement surfaces respectively slidingly engage with said inner control surfaces to move said pair of jaw arms toward each other.

4. The mechanism according to claim 1, wherein each said inner engagement surface and said outer engagement surface are discontinuous with each other.

5. The mechanism according to claim 1, wherein:
each said jaw arm further comprises a second inner engagement surface;
said cinch comprises a central block having opposed ends, each end of said opposed ends configured to contact a respective second inner engagement surface to open said jaw arms when said cinch moves rearwardly along the longitudinal axis.

6. The mechanism according to claim 5, wherein:
each said inner engagement surface and each said outer engagement surface are located on the same surface of a respective jaw arm; and
each said second inner engagement surface are located on a surface of a respective jaw arm that is opposite to the jaw arm surface on which said each said inner engagement surface and said each said outer engagement surface are located.

7. The mechanism according to claim 1, wherein:
said pair of control members is a pair of control fingers;
a leading edge of each control finger of said pair of control fingers is affixed to a distal end of said cinch and forms said inner control surface;
a trailing edge of each said control finger forms said outer control surface.

8. The mechanism according to claim 7, wherein at least a portion of each jaw arm is sandwiched between an underside of said cinch and a respective said control finger.

9. The mechanism according to claim 1, wherein said cinch further comprises a pair of inwardly facing guide walls configured to respectively slidingly engage at least a portion of an outer side region of said pair of jaw arms during movement of said cinch along the longitudinal axis.

10. The mechanism according to claim 1, wherein said jaw arms are biased to an open position.

11. A method of opening and closing jaws of a medical instrument, the instrument including a cinch movable between a first position and a second position, and to an intermediate position located between the first and second positions, the cinch having a pair of control members, each control member having an inner control surface and an outer control surface, the instrument further including a jaw assembly having a pair of jaw arms, each jaw arm comprising an inner engagement surface and an outer engagement surface, the method comprising:
moving the cinch along a longitudinal axis of the instrument, from the intermediate position toward the first position such that the inner engagement surfaces slidingly engage with the respective outer control surfaces to one of move the jaws together or move the jaws apart; and
moving the cinch along the axis from the intermediate position toward the second position such that the outer engagement surfaces slidingly engage with the respective inner control surfaces to the other of move the jaws together or move the jaws apart.

12. The method according to claim 11, wherein each inner engagement surface and each outer engagement surface are discontinuous with each other.

13. The method according to claim 11, wherein each said jaw arm further comprises a second inner engagement surface, and wherein said cinch comprises a central block having opposed ends, the method further comprising moving the cinch along the axis toward one of the first or second positions such that each opposed end of the central block contacts a respective second inner engagement surface to open said jaw arms.

14. The method according to claim 13, wherein:
each said inner engagement surface and each said outer engagement surface are located on the same surface of a respective jaw arm; and
each said second inner engagement surface are located on a surface of a respective jaw arm that is opposite to the jaw arm surface on which said each said inner engagement surface and said each said outer engagement surface are located.

15. The method according to claim 11, wherein:
said pair of control members is a pair of control fingers;
a leading edge of each control finger of said pair of control fingers is affixed to a distal end of said cinch and forms said inner control surface;
a trailing edge of each said control finger forms said outer control surface.

16. The method according to claim 15, further comprising sandwiching at least a portion of each jaw arm between an underside of said cinch and a respective said control finger.

17. The method according to claim 11, wherein said cinch further comprises a pair of inwardly facing guide walls, the method further comprising moving the cinch along the axis such that at least a portion of respective outer side regions of said pair of jaw arms respectively slidingly engage the pair of guide walls.

18. The method according to claim 11, further comprising biasing said jaw arms to an open position.

19. The method according to claim 11, wherein:
the first position is a proximal position;
the second position is a distal position;
said moving the cinch from the intermediate position toward the first position moves the jaws apart; and
said moving the cinch from the intermediate position toward the second position moves the jaws together.

20. A clip applying device for crimping a clip onto tissue, comprising:
a body assembly having a handle and a squeezable trigger;
a barrel having a first end extending into said body assembly;
a cinch configured to move rearwardly and forwardly along a longitudinal axis, said cinch comprising a pair of control members each having an inner control surface and an outer control surface
a pair of jaw arms arranged on a second end of said barrel, said jaws actuable by movement of said cinch in response to action of said trigger, each jaw arm comprising an inner engagement surface configured to slidingly engage with a respective one of said outer control surfaces, and an outer engagement surface configured to slidingly engage with a respective one of said inner control surfaces, said second end of said barrel and at least a portion of said pair of jaw arms configured to be inserted into a body cavity.

21. The device according to claim 20, wherein:
each said jaw arm further comprises a second inner engagement surface;
said cinch comprises a central block having opposed ends, each end of said opposed ends configured to contact a respective second inner engagement surface to open said jaw arms when said cinch moves rearwardly along the longitudinal axis.
